(19)

**Europäisches Patentamt**
**European Patent Office**
**Office européen des brevets**

(11) **EP 3 438 227 B1**

(12) **EUROPEAN PATENT SPECIFICATION**

(45) Date of publication and mention
of the grant of the patent:
**06.11.2024 Bulletin 2024/45**

(21) Application number: **17775068.4**

(22) Date of filing: **28.03.2017**

(51) International Patent Classification (IPC):
*C09K 11/02* (2006.01)    *C09K 11/77* (2006.01)
*C09K 11/06* (2006.01)    *A61K 9/51* (2006.01)
*G01N 21/64* (2006.01)    *A61K 41/00* (2020.01)
*A61K 47/02* (2006.01)    *A61P 35/00* (2006.01)
*B82Y 5/00* (2011.01)     *B82Y 20/00* (2011.01)
*B82Y 30/00* (2011.01)    *G01N 33/48* (2006.01)

(52) Cooperative Patent Classification (CPC):
**C09K 11/06; A61K 9/51; A61K 41/00; A61K 47/02;**
**A61P 35/00; B82Y 5/00; B82Y 20/00; B82Y 30/00;**
**C09K 11/02; C09K 11/025; C09K 11/771;**
**C09K 11/77342; G01N 33/48;** G01N 21/6408;
G01N 21/6458;                                     (Cont.)

(86) International application number:
**PCT/JP2017/012605**

(87) International publication number:
**WO 2017/170531 (05.10.2017 Gazette 2017/40)**

(54) **LIGHT-EMITTING NANOPARTICLES, CELL DETECTION METHOD USING SAME, ANIMAL TREATMENT METHOD, MEDICAL DEVICE, CELL VISUALIZATION METHOD, AND METHOD FOR REDUCING INJURY TO CELL**

LICHTEMITTIERENDE NANOPARTIKEL, ZELLDETEKTIONSVERFAHREN DAMIT, TIERBEHANDLUNGSVERFAHREN, MEDIZINISCHE VORRICHTUNG, ZELLVISUALISIERUNGSVERFAHREN UND VERFAHREN ZUR REDUZIERUNG VON VERLETZUNGEN AN ZELLEN

NANOPARTICULES ÉLECTROLUMINESCENTES, PROCÉDÉ DE DÉTECTION DE CELLULES L'UTILISANT, PROCÉDÉ DE TRAITEMENT D'ANIMAUX, DISPOSITIF MÉDICAL, PROCÉDÉ DE VISUALISATION DE CELLULES ET PROCÉDÉ POUR RÉDUIRE UNE LÉSION À UNE CELLULE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.03.2016 JP 2016064240**

(43) Date of publication of application:
**06.02.2019 Bulletin 2019/06**

(73) Proprietors:
• **Ohara, Inc.**
**Sagamihara-shi, Kanagawa 252-5286 (JP)**
• **National University Corporation Nagaoka University**
**Nagaoka-shi, Niigata 940-2188 (JP)**

• **National Institute of Technology**
**Hachioji-shi, Tokyo 193-0834 (JP)**

(72) Inventors:
• **INUI, Masahiko**
**Wakayama-shi**
**Wakayama 640-8404 (JP)**
• **CHATANI, Sunao**
**Wakayama-shi**
**Wakayama 640-8404 (JP)**
• **TAGAYA, Motohiro**
**Nagaoka-shi**
**Niigata 940-2188 (JP)**
• **MOTOZUKA, Satoshi**
**Motosu-shi**
**Gifu 501-0495 (JP)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(74) Representative: **Maiwald GmbH**
Elisenhof
Elisenstraße 3
80335 München (DE)

(56) References cited:

- MOTOHIRO TAGAYA ET AL: "Synthesis of Luminescent Nanoporous Silica Spheres Functionalized with Folic Acid for Targeting to Cancer Cells", INORGANIC CHEMISTRY, vol. 53, no. 13, 12 June 2014 (2014-06-12), EASTON, US, pages 6817 - 6827, XP055430105, ISSN: 0020-1669, DOI: 10.1021/ic500609g
- CHEN, XIAOHU ET AL: "Large-scale synthesis of water-soluble luminescent hydroxyapatite nanorods for security printing", JOURNAL OF COLLOID AND INTERFACE SCIENCE, vol. 468, 1 February 2016 (2016-02-01), pages 300 - 306, XP002794409
- KOTA SHIBA ET AL: "Synthesis of Cytocompatible Luminescent Titania/Fluorescein Hybrid Nanoparticles", ACS APPLIED MATERIALS & INTERFACES, vol. 6, no. 9, 25 April 2014 (2014-04-25), US, pages 6825 - 6834, XP055430110, ISSN: 1944-8244, DOI: 10.1021/am500636d
- MOTOHIRO TAGAYA ET AL: "Synthesis and luminescence properties of Eu(III)-doped nanoporous silica spheres", JOURNAL OF COLLOID AND INTERFACE SCIENCE, ACADEMIC PRESS,INC, US, vol. 363, no. 2, 17 July 2011 (2011-07-17), pages 456 - 464, XP028288398, ISSN: 0021-9797, [retrieved on 20110805], DOI: 10.1016/J.JCIS.2011.07.066
- MOTOHIRO TAGAYA ET AL: "Efficient synthesis of Eu(III)-containing nanoporous silicas", MATERIALS LETTERS, ELSEVIER, AMSTERDAM, NL, vol. 65, no. 14, 3 April 2011 (2011-04-03), pages 2287 - 2290, XP028387401, ISSN: 0167-577X, [retrieved on 20110412], DOI: 10.1016/J.MATLET.2011.04.011
- TAGAYA MOTOHIRO ET AL: "In Vitro Targeting of Cancer Cells with Luminescent Nanoporous Silica Spheres", TRANSACTIONS ON GIGAKU, NAGAOKA UNIVERSITY OF TECHNOLOGY, NIIGATA, vol. 1, no. 1014, 30 June 2012 (2012-06-30), pages 1 - 8, XP009514948
- ROSTICHER CÉLINE ET AL: "Persistent luminescence of Eu, Mn, Dy doped calcium phosphates for in-vivo optical imaging", JOURNAL OF LUMINESCENCE, ELSEVIER BV NORTH-HOLLAND, NL, vol. 170, 29 July 2015 (2015-07-29), pages 460 - 466, XP029330954, ISSN: 0022-2313, DOI: 10.1016/J.JLUMIN.2015.07.024
- ZHANG C ET AL: "Self-activated luminescent and mesoporous strontium hydroxyapatite nanorods for drug delivery", BIOMATERIALS, ELSEVIER SCIENCE PUBLISHERS BV., BARKING, GB, vol. 31, no. 12, 1 February 2010 (2010-02-01), pages 3374 - 3383, XP027575380, ISSN: 0142-9612, [retrieved on 20100201]
- TAGAYA, M. ET AL.: "Synthesis of Luminescent Nanoporous Silica Spheres Functionalized with Folic Acid for Targeting to Cancer Cells", INORGANIC CHEMISTRY, vol. 53, 2014, pages 6817 - 6827, XP055430105
- TAGAYA, M. ET AL.: "In Vitro Targeting of Cancer Cells with Luminescent Nanoporous Silica Spheres", TRANSACTIONS ON GIGAKU, vol. 1, no. 01014, 2012, pages 1 - 8, XP009514948
- TAGAYA, M. ET AL.: "Synthesis and Luminescence Properties of Eu(III)-doped Nanoporous Silica Spheres", JOURNAL OF COLLOID AND INTERFACE SCIENCE, vol. 363, no. 2, 2011, pages 456 - 464, XP028288398
- TAGAYA, M. ET AL.: "Efficient Synthesis of Eu (III)-Containing Nanoporous Silicas", MATERIALS LETTERS, vol. 65, no. 14, 2011, pages 2287 - 2290, XP028387401
- MOTOHIRO TAGAYA: "Koji Kozo Seigyo sareta Muki/ Yuki Nano Fukugotai no Sosei to Hikari Kinoka", DV-XA KENKYU KYOKAI KAIHO, vol. 26, no. 1 to 2, 2014, pages 22 - 26, XP009516000, ISSN: 1346-5015
- SHIBA, K. ET AL.: "Synthesis of Cytocompatible Luminescent Titania/Fluorescein Hybrid Nanoparticles", ACS APPLIED MATERIALS & INTERFACES, vol. 6, no. 9, 2014, pages 6825 - 6834, XP055430110
- SHIBA, K. ET AL.: "Preparation of Luminescent Titania/Dye Hybrid Nanoparticles and Their Dissolution Properties for Controlling Cellular Environments", RSC ADVANCES, vol. 5, no. 5, 2015, pages 104343 - 104353, XP055430114
- RYUHEI TAKEDA ET AL.: "Eu Tenka Apatite Ryushi no Gosei to Hakko Tokusei", DV-XQI KENKYUKAI KAIHO, vol. 24, no. 1-2, 2012, pages 243 - 246, XP009515994, ISSN: 1346-5015

(52) Cooperative Patent Classification (CPC): (Cont.)
G01N 2223/3307

**Description**

TECHNICAL FIELD

The present invention relates to a light emitting nanoparticle. Particularly preferably, the present invention relates to a light emitting nanoparticle used for bioimaging.

BACKGROUND ART

Recently, there has been demand for a technique of detecting with high sensitivity at the cell level, tumors such as cancer cells and the like, by a marker material having a high biocompatibility.

Patent Document 1 discloses, as a bioimaging material, a two-photo absorbing material consisting of a water soluble dendrimer wherein a dye having a two photon absorbing-property and a dendron are bonded.

Patent Document 2 discloses the use of a water-dispersible quantum dot wherein a surface of the quantum dot is coated with a surfactant-type polymerization initiator comprising a hydrophobic group and a polar group, as a particle for in-vivo bioimaging.

Patent Document 3 discloses a production method of a bioimaging nanoparticle comprising the step of producing a hydrophobic nanoparticle which maintains individual dispersibility in a nonpolar organic solvent, wherein in a hydrophobic inorganic nanoparticle having a core or core/shell structure protected by a surfactant, the surfactant is partially substituted by adding 1 to 30 equivalents of an organic ligand wherein a thiol group and a hydrophilic group are bonded by a hydrocarbon chain with a carbon number of 8 to 20, and the surface of the nanoparticles is surface-modified so that only one part is hydrophilic, by forming a metal thiolate (M-S) bond, and the like.

Patent Document 4 discloses a fluorescent particle used for bioimaging, which is a fluorescent particle having upconversion characteristics which is a phenomenon which uses a low energy light such as an infrared light or the like as an excitation light, to obtain visible light fluorescence, and the material of the fluorescent particles is one material, or a combination of two or more materials among $Y_2O_2$:$Er^{3+}$, $Yb^{3+}$, $Y_2O_3$:$Er^{3+}$, $NaYF_4$:$Er^{3+}$, $Yb^{3+}$.

Patent Document 5 discloses a semiconductor nanoparticle used for molecule/cell imaging, which is a semiconductor nanoparticle with an average particle diameter of 1 to 20 nm, comprising an atom pair a main component element constituting the same, and an atom pair of the corresponding differing atom or a differing atom which has an equivalent valence electron arrangement, and further the dopant is distributed on the semiconductor nanoparticle surface or the vicinity thereof.

Patent Document 6 discloses a fluorescent labeling agent for pathological diagnosis comprising fluorescent material encapsulated nanoparticles comprising a first fluorescent material, and a second fluorescent material having a distinguishable excitation/luminescence property from the first fluorescent material.

Patent Document 7 discloses a fluorescent labeling agent, and a kit for target molecule measurement, consisting of a rare earth fluorescent complex-comprising silica particle comprising a rare earth fluorescent complex.

Further, Non-Patent Documents 1 to 4 also disclose the use of quantum dots and fluorescent dyes for bioimaging. Non-Patent Document 5 discloses luminescent europium(III)-doped nanoporous silica nanospheres. Non-Patent Document 6 discloses luminescent $Eu^{3+}$-doped hydroxyapatite nanorods for the preparation of fluorescent inks. Non-Patent Document 7 discloses the synthesis of cytocompatible luminescent titania/fluorescin hybrid nanoparticles. Non-Patent Document 8 discloses the synthesis and luminescence properties of Eu(III)-doped nanoporous silica spheres. Non-Patent Document 9 relates to Eu(III)-containing nanoporous silica, which was synthesized by two different loadings utilizing adsorption and doping. Non-Patent Document 10 describes luminescent Eu(III)-doped nanoporous silica spheres by a supramolecular templating method. Non-Patent Document 11 relates to calcium phosphate nanoparticles doped with $Eu^{3+}$, $Mn^{2+}$ and $Ln^{3+}$ ions synthesized by a hydrothermal route. Non-patent document 12 describes strontium hydroxyapatite nanorods with luminescent and mesoporous properties synthesized by a hydrothermal method.

Patent Document 1: Japanese Unexamined Patent Application, Publication No. 2010-133887
Patent Document 2: Japanese Unexamined Patent Application, Publication No. 2009-190976
Patent Document 3: Japanese Unexamined Patent Application, Publication No. 2009-107106
Patent Document 4: Japanese Unexamined Patent Application, Publication No. 2013-14651
Patent Document 5: PCT International Publication No. WO2009/066548
Patent Document 6: Japanese Unexamined Patent Application, Publication No. 2013-57037
Patent Document 7: PCT International Publication No. WO2005/023961
Non-Patent Document 1: "Selective molecular imaging of viable cancer cells with pH-activatable fluorescence probes " Nature Medicine 15, 104 (2009)
Non-Patent Document 2: "Quantum Dot Bioconjugates for Ultrasensitive Nonisotopic Detection." Science 281, 2016 (1998)

Non-Patent Document 3: "Nucleic Acid-Passivated Semiconductor Nanocrystals: Biomolecular Templating of Form and Function." Accounts of Chemical Research, 43, 173 (2010)

Non-Patent Document 4: "Multimodal-Luminescence Core-Shell Nanocomposites for Targeted Imaging of Tumor Cells." Chem. Eur. J., 15, 3577 (2009)

Non-Patent Document 5: "Synthesis of Luminescent Nanoporous Silica Spheres Functionalized with Folic Acid for Targeting to Cancer Cells." Inorganic Chemistry, vol. 53, no.13, 12 June 2014, pages 6817-6827

Non-Patent Document 6: "Large-Scale Synthesis of Water-Soluble Luminescent Hydroxyapatite Nanorods for Security Printing." Journal of Colloid and Interface Science, vol. 468, 1 February 2016, pages 300-306

Non-Patent Document 7: "Synthesis of Cytocompatible Luminescent Titania/Fluorescin Hybrid Nanoparticles". ACS Applied materials & Interfaces, vol. 6, no. 9, 25 April 2014, pages 6825-6834

Non-Patent Document 8: "Synthesis and Luminescence Properties of Eu(III)-doped Nanoporous Silica Spheres". Journal of Colloid and Interface Science, Academic Press, Inc., US, vol. 363, no.2, 17 July 2011, pages 456-464

Non-Patent Document 9: "Efficient Synthesis of Eu(III)-Containing Nanoporous Silicas". Materials Letters, Elsevier, Amsterdam, NL, vol. 65, no. 14, 3 April 2011, pages 2287-2290

Non-Patent Document 10: "In Vitro Targeting of Cancer Cells with Luminescent Nanoporous Silica Spheres". Transactions on Gigaku, Nagaoka University of technology, Niigata, vol. 1, no. 1014, 30 June 2012, pages 1-8

Non-Patent Document 11: "Persistent Luminescence of Eu, Mn, Dy-doped Calcium Phosphates for in-Vivo Optical Imaging". Journal of Luminescence, Elsevier BV North-Holland, NL, vol. 170, 29 July 2015, pages 460-466.

Non-Patent Document 12: "Self-Activated Luminescent and Mesoporous Strontium Hydroxyapatite Nanorods for Drug Delivery". Biomaterials, Elsevier Science Publishers BV, Barking, GB, vol. 31, no. 12, 1 February 2010, pages 3374-3383.

DISCLOSURE OF THE INVENTION

Problems to be Solved by the Invention

As described above, in recent years, there has been demand for a technique for high sensitivity detection of tumors such as cancer cells or the like at the cellular level by a marker having high biocompatibility. For example, in the canceration of a cell, before a morphological change occurs, an operational change at the molecular level occurs. For example, a cancer cell tends to consume a large amount of dextrose compared to a normal cell. At the same time, because folate receptors are overexpressed on the cell membrane, there is a tendency to selectively bond/acquire folic acid molecules. If high sensitivity imaging of such changes at the molecular level of a cell were possible, it would become possible to realize very early stage detection of cancer cells and the like.

However, as an imaging material for imaging, in the case of using an organic molecule, the rate of degradation/fading is high, for example, there has been the problem that light-emitting particles become quenched or the like by photoirradiation after several tens of minutes under fluorescence observation. Further, as a bioimaging material, in the case of using an inorganic material such as quantum dots or the like, in some cases, highly poisonous elements such as cadmium or the like are included, and there have been problems with biocompatibility and the like.

The present invention has the objective of providing light-emitting nanoparticles, which is provided with light-emitting stability and light resistance, and is low in biological toxicity, and a cell detection method, treatment method of animals, medical device, a cell visualization method, and a damage reduction method of cells using the same.

Means for Solving the Problems

The present inventors, in order to achieve the above objective, as a result of diligent research, created a composite particle wherein a light-emitting molecule or ion is included in an inorganic material, and invented a light-emitting nanoparticle which is provided with light emitting stability and light resistance, and is low in biological toxicity, and a cell detection method, treatment method of animals, medical device, cells visualization method, and damage reduction method of cells using the same.

The invention is set out in the appended set of claims.

Effects of the Invention

According to the present invention, it is possible to provide a light-emitting nanoparticle, which is provided with light-emitting stability and light resistance, and is low in biological toxicity, and a cell detection method, treatment method of animals, medical device, a cell visualization method, and a damage reduction method of cells using the same.

BRIEF DESCRIPTION OF THE DRAWINGS

Fig. 1 is a schematic diagram showing the outer appearance of the light-emitting nanoparticles according to one embodiment of the present invention.

Fig. 2 is a diagram schematically showing the mechanism whereby the light-emitting nanoparticles according to one embodiment of the present invention are taken up by cancer cells.

Fig. 3 is a transmission electron microscope (TEM) image showing the distribution of the light-emitting substance in each type of base material.

Fig. 4 is a graph showing the relationship between the light-emitting substance concentration in each matrix material and the fluorescence lifetime ($\tau$).

Fig. 5 is a transmission electron microscope (TEM) image showing the concentration distinction of the $Eu^{3+}$-containing silica particles.

Fig. 6 is a graph showing the nitrogen adsorption/desorption isotherm and the pore distribution curve for the case that the matrix material is silica.

Fig. 7 is a particle size distribution and electron microscope-observed image (FE-SEM) according to concentration of fluorescein isothiocyanate (FITC)-containing titania particles.

Fig. 8 is an electron microscope-observed image (TEM) according to concentration of $Eu^{3+}$-containing calcium phosphate compound particles.

Fig. 9 is a graph showing X-ray diffraction patterns, (a) $Eu^{3+}$-containing silica particles, (b) FITC-containing titania particles, and (c) $Eu^{3+}$-containing calcium phosphate compound particles.

Fig. 10 is a graph showing the infrared absorption spectrum before surfactant removal for the $Eu^{3+}$-containing silica particles.

Fig. 11 is a graph showing the infrared absorption spectrum, (a) $Eu^{3+}$-containing silica particles, (b) FITC-containing titania particles, and (c) $Eu^{3+}$-containing calcium phosphate compound particles.

Fig. 12 is a graph showing an excitation spectrum, (a) $Eu^{3+}$-containing silica particles, (b) FITC-containing titania particles, and (c) $Eu^{3+}$-containing calcium phosphate compound particles.

Fig. 13 is a graph showing an emission spectrum, (a) $Eu^{3+}$-containing silica particles, (b) FITC-containing titania particles, and (c) $Eu^{3+}$-containing calcium phosphate compound particles.

Fig. 14 is a graph showing the intensity spectra of the incident light, scattered light, and fluorescent light.

Fig. 15 is a graph showing the results of cytotoxic quantification, (a) light-emitting nanoparticles which are not modified by folic acid bonding to cells, (b) light-emitting nanoparticles which are modified by folic acid bonding to cells.

Fig. 16 is, for $Eu^{3+}$-containing silica particles differing by having or not having modification of cell bonding molecules, and (a) is a graph showing the relationship between fluorescent light intensity and culture time, and (b) and (c) are fluorescence imaging images of cells which have taken up the particles.

Fig. 17 is, for FITC-containing titania particles differing by having or not having modification of cell bonding molecules, and (a) is a graph showing the relationship between fluorescent light intensity and culture time, and (b) and (c) are fluorescence imaging images of cells which have taken up the particles.

Fig. 18 is, for $Eu^{3+}$-containing calcium phosphate compound particles differing in having or not having modification of cell bonding molecules, and (a) is a graph showing the relationship between fluorescent light intensity and culture time, and (b) and (c) are fluorescence imaging images of cells which have taken up the particles.

PREFERRED MODE FOR CARRYING OUT THE INVENTION

Below, examples of the invention are described. Further, the present invention is not to be interpreted as being limited by these examples.

The first light-emitting nanoparticle of the present examples comprise a matrix material, and a light-emitting substance included in the matrix material. The matrix material comprises at least one type of cationic element selected from the group consisting of Ti, Si, Ca, Al and Zr, and surfactant molecule, and at least one type of anionic element selected from the group consisting of O and P.

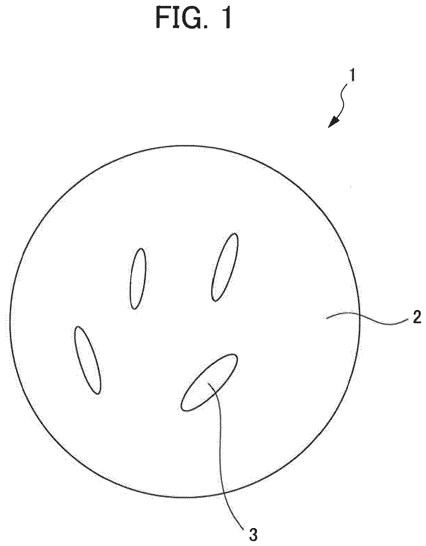

Fig. 1 is a schematic diagram schematically showing the outer appearance of the first light-emitting nanoparticle 1 of the present example. The first light-emitting nanoparticle 1 is provided with the matrix material 2, and the light-emitting substance 3 is included in the matrix material 2.

In the first light-emitting nanoparticle 1, the concentration of the light-emitting substance in the matrix material is a concentration whereby the average distance between the light-emitting substance becomes 1.2 nm or more. Herein, the "average distance between the light-emitting substance" is a theoretical value derived by calculating as below, from the average particle diameter of the light-emitting particles, the density, the molecular weight of the matrix material, the concentration of the light-emitting substance and the like. Accordingly, "the concentration of the light-emitting substance in the matrix material is a concentration whereby the average distance between the light-emitting substance becomes 1.2 nm or more" is a concentration set such that the theoretical average distance of the light-emitting substance, derived by calculations using formulas (1) to (6), becomes 1.2 nm or more.

Furthermore, when calculating the average distance of the light-emitting substance, it is confirmed that the light-emitting substance is in a state of approximately uniformly distribution in the light-emitting nanoparticle. The confirmation of the distribution state of the light-emitting substance can be carried out by transmission electron microscopy (TEM). Further, it can also be confirmed that the light-emitting substance is in a state of approximately uniformly distribution according to the fluorescence life measurement method, from the relationship between the light-emitting substance concentration and the fluorescence life. In a plurality of light-emitting nanoparticles where the concentrations of the light-emitting substance differ, when the light-emitting substance in the respective light-emitting nanoparticles is approximately uniform, a plot of the fluorescence life vs each concentration of the light-emitting substance displays a correlation with a negative linearity. This is because, as the concentration of the light-emitting substance increases, the occupied volume contributed by the light-emitting substance linearly decreases, the distance between the light-emitting substance becomes shorter, and the rate of the cross-relaxation process becomes higher. If the light-emitting substance aggregates, such a correlation does not occur. Accordingly, according to the fluorescence life measurement method, in order to confirm whether or not the light-emitting substance in the light-emitting nanoparticle is approximately uniform without agglomerating, the following procedure may be used. First, the concentration of the constituent components of these light-emitting nanoparticles and the light-emitting substance are analyzed, and a plurality of samples having differing concentrations of the light-emitting substance are prepared, consisting of the same constituent components as these light-emitting nanoparticles. Next, the fluorescence life of the plurality of samples is measured, and it is confirmed that the relationship between the concentration and the fluorescence life is a linear correlation. For the plurality of samples, if the relationship between the concentration of the light-emitting substance and the fluorescence life can be confirmed to be a linear correlation, after this, the fluorescence life of the light-emitting nanoparticle which is the subject of analysis is measured, and it is confirmed whether or not in the relationship between the concentration of the light-emitting substance and the fluorescence life, the plot of the sample and analysis is a linear correlation. If the plot of the sample and analysis is a linear correlation, it can be confirmed that the light-emitting substance in the light-emitting nanoparticle is approximately uniformly distributed.

The average particle diameter of the light-emitting nanoparticle is the averaged value of a plurality of particles obtained by determining the particle diameter of the light-emitting nanoparticle as {(long diameter + short diameter)/2}. For example, it is preferable to calculate the average particle diameter for 100 arbitrary particles in a predetermined region, using a scanning electron microscope (FE-SEM).

Specifically, the average distance between the light-emitting substance can be calculated from the following formulas (1) to (6).

(Inorganic Molecular Weight Density)

$$De = A \cdot \rho_n/M \quad Formula\ (1)$$

De: inorganic molecular number density (number of molecules/nm$^3$)
A: Avogadro's number ($6.02 \times 10^{23}$)
$\rho_n$: nm density (g/nm$^3$)
M: each molecular weight (silica (S): 79.866, titania (T) 60.1, hydroxyapatite (CP): 1004.62)

(Average One Particle Volume)

$$V_1 = (4\pi/3) \cdot (R/2)^3 \quad Formula\ (2)$$

$V_1$: average one particle volume (nm$^3$/1 particle)
R: average particle diameter (nm)

(Contained Inorganic Molecule Number)

$$X_1 = De \cdot V_1 \quad \text{Formula (3)}$$

$X_1$: inorganic molecule number contained in the light-emitting nanoparticle (molecule number/1 particle)

(Contained Light-Emitting Substance Number)

$$X_2 = X_1 \cdot B \quad \text{Formula (4)}$$

$X_2$: light-emitting substance number contained in the light-emitting nanoparticle (molecule number/1 particle)
B: inorganic molecule number per light-emitting substance number (molecule number/substance number)

(Occupied Volume of 1 Light-Emitting Substance Molecule)

$$V_2 = V_1/X_2 \quad \text{Formula (5)}$$

$V_2$: occupied volume of 1 molecule of light-emitting substance ($nm^3$/1 molecule of light-emitting substance)

(Distance Between Light-Emitting Substance)

$$D = \{V_2 \cdot 3/(4\pi)\}^{1/3} \quad \text{Formula (6)}$$

D: distance between centers of light emitting substance (nm)

When the average distance between the light-emitting substance is 1.2 nm or more, it is possible to prevent density quenching caused by agglomerates of the light-emitting substance 4, and the light-emitting nanoparticles have excellent light emission stability. On the other hand, the present inventors surmise that if the average distance between the light-emitting substance is short, because of behavior as an excited complex, the energy occurring when excited is transferred and does not become light-emission energy, and light-emission rate reduction and the like occurs. The average distance between the light-emitting substance is preferably 1.5 nm or more, and more preferably 2 nm or more. Further, the upper limit of the average distance between the light-emitting substance is preferably 10 nm or less from the viewpoint of the sensitivity of general purpose fluorescence detection equipment. The concentration of the light-emitting substance in the matrix material corresponds to the light-emitting substance number B with respect to the inorganic molecule number. Therefore, a concentration where the average distance between the light-emitting substance is 1.2 nm or more can be determined depending on the used each molecular weight M and average particle diameter R according to the above equations (1) to (6) .

The matrix material comprises at least one type of cationic element selected from the group consisting of Ti, Ca, Al, and Zr. As a suitable matrix material, titanium oxide comprising Ti which is a cationic element and O which is an anionic element, or silicon oxide comprising Si which is a cationic element, and O which is an anionic element, or aluminum oxide comprising Al which is a cationic element and O which is an anionic element, or zirconium oxide comprising Zr which is a cationic element and O which is an anionic element, may be mentioned.

The titanium oxide and silicon oxide may be particles of metal alkoxides shown by the general formula $M(OR)_n$ (M is Si or Ti, R is an alkyl group with a carbon number of 1 to 5) are agglomerated by hydrogen bonds, or may be particles wherein metal alkoxides are dehydration condensed with each other, to form a skeleton structure ($-(M-O-M)_n-$).

As the matrix material, a calcium phosphate comprising Ca which is a cationic element and P which is an anionic element may also be mentioned.

The calcium phosphate compound is preferably a mixed compound of a phosphoric oxide source (one or more types of salt selected from the group of phosphoric oxide, monosodium phosphate, disodium phosphate, monocalcium phosphate, dicalcium phosphate, monoantimony phosphate, diantimony phosphate, and the like), and a calcium source (one or more types salt selected from the group of calcium nitrate, calcium carbonate, calcium chloride, calcium hydroxide, calcium acetate, and the like), or mixed reactants. As the calcium phosphate compound, calcium monohydrogen phosphate anhydride ($CaHPO_4$), calcium monohydrogen phosphate dihydride ($CaHPO_4 \cdot 2H_2O$), tricalcium phosphate ($Ca_3(PO_4)_2$), dihydrogen calcium phosphate anhydride ($Ca(H_2PO_4)_2$), calcium dihydrogen phosphate hydride ($Ca(H_2PO_4)_2 \cdot H_2O$), tetracalcium phosphate ($Ca_4O(PO_4)_2$), hydroxyapatite ($Ca_{10}(PO_4)_6(OH)$), octacalcium phosphate ($Ca_8H_2(PO_4)_6 \cdot 5H_2O$), amorphous calcium phosphate ($Ca_3(PO_4)_2 \cdot nH_2O$) may be mentioned.

In the first light-emitting nanoparticle, the matrix material preferably comprises at least one type selected from the group consisting of $TiO_2$, $SiO_2$, $Ca_{10}(PO_4)_6(OH)_2$, $Al_2O_3$, and $ZrO_2$. These matrix materials have low toxicity towards living bodies, and are excellent in biocompatibility. Among these, $TiO_2$, $SiO_2$, and $Ca_{10}(PO_4)_6(OH)_2$ are preferable as the matrix material of the first light-emitting nanoparticle.

The second light-emitting nanoparticle of the present embodiment is a light-emitting nanoparticle comprising a matrix material, and a light-emitting substance included in the matrix material, and the matrix material comprises at least one type of cationic element selected from the group consisting of Ti, Ca, Al, and Zr, and at least one type of anionic element selected from the group consisting of O and P.

In the second light-emitting nanoparticle, the matrix material preferably comprises at least one type selected from the group consisting of $TiO_2$, $Ca_{10}(PO_4)_6(OH)_2$, $Al_2O_3$, and $ZrO_2$. These matrix materials have low toxicity towards living bodies, and are excellent in biocompatibility. Among these, $TiO_2$ and $Ca_{10}(PO_4)_6(OH)_2$ are more preferable as the matrix material of the second light-emitting nanoparticle.

Also in the second light-emitting nanoparticle, it is preferable if the average distance between the light-emitting substance is 1.2 nm or more, because it is possible to prevent concentration quenching of the light-emitting substance, and it becomes possible to obtain light-emitting nanoparticle with excellent light-emission stability. The average distance between the light-emitting substance is preferably 1.5 nm or more, and more preferably 2 nm or more. Further, the upper limit of the average distance between the light-emitting substance is preferably 10 nm or less from the viewpoint of the sensitivity of general purpose fluorescence detection equipment.

In the first light-emitting nanoparticle or the second light-emitting nanoparticle (below, when the first light-emitting nanoparticle as well as the second light-emitting nanoparticle are the subject, these may be referred to simply as "light-emitting nanoparticle"), the light-emitting substance preferably comprises at least one type selected from the group consisting of an organic light-emitting dye and a rare earth ion.

The organic light-emitting dye is not particularly limited, but is preferably at least one type selected from the group consisting of fluoresceine-based dye molecules, rhodamine-based dye molecules, cascade system dye molecules, coumalin-based dye molecules, eosin-based dye molecules, pyrene-based dye molecules, and cyanine-based dye molecules. Among these, fluoresceine-based dye molecules are preferable, and for example, fluorescein isothiocyanate (FITC) is excited and emits in the visible light region, and therefore is suitably used as the light-emitting substance in the present examples.

The rare earth ion is preferably at least one type selected from the group consisting of trivalent Ce, tetravalent Ce, trivalent Pr, trivalent Nd, trivalent Pm, trivalent Sm, divalent Eu, trivalent Eu, trivalent Gd, trivalent Tb, trivalent Dy, trivalent Ho, trivalent Er, trivalent Tm, trivalent Yb, and trivalent Lu. Among these, $Eu^{3+}$ which is trivalent Eu is excited and emits in the visible light region, and therefore is suitably used as the light-emitting substance in the light-emitting nanoparticles of the present examples.

The contained concentration of the organic light-emitting dye is preferably 1 mmol% to 6 mol% with respect to the cationic element of the matrix material. When the contained concentration of the organic light-emitting dye is within this range, there is a tendency to readily maintain an average distance between the light-emitting substance of 1.2 nm or more. The contained concentration of the organic light-emitting dye is more preferably 100 mmol% to 5.5 mol%, and even more preferably 1 mol% to 5 mol%, with respect to the cationic element. The contained concentration of the rare earth ion is preferably 1 mmol% to 10 mol% with respect to the cationic element of the matrix material. When the contained concentration of the rare earth ion is within this range, there is a tendency to readily maintain an average distance between the light-emitting substance of 1.2 nm or more. The contained concentration of the rare earth ion is more preferably 100 mmol% to 10 mol%, and even more preferably 1 mol% to 5 mol%, with respect to the cationic element.

The matrix material preferably comprises a surfactant molecule. By comprising a surfactant molecule, there is a tendency to readily maintain a suitable average distance between the light-emitting substances. The surfactant molecule is not particularly limited, and for example, hexadecyltrimethylammonium bromide (tyltrimethylammonium bromide), hexadecyl trimethyl ammonium chloride, octadecyl trimethyl ammonium bromide, decyltrimethylammonium bromide, dodecyltrimethylammonium bromide, hexadecyl dimethyl ethyl ammonium bromide, hexadecylamine, sodium dodecyl sulfate, hexadecylamine, octadecylamine, octylphenol ethoxylate, polyoxyethylene cetyl ether, polyoxyethylene lauryl ether, polyoxyethylene(EO)-polyoxypropylene glycol (PO) copolymer ($EO_{20}PO_{30}$), polyoxyethylene (EO)-polyoxypro-

pylene glycol (PO)-polyoxyethylene (EO) amphiphilic triblock copolymer ($EO_5PO_{68}EO_5$, $EO_{20}PO_{70}PEO_{20}$, $EO_{97}PO_{67}EO_{97}$) and the like, may be used. Further, in the case that it is not required to maintain a suitable average distance between the light-emitting substance, it is not necessary for the matrix material to comprise a surfactant molecule.

The content of the surfactant molecule in the matrix material is preferably a mol ratio of 0.01 or more with respect to the metal element of the matrix material. By making the mol ratio 0.01 more with respect to the metal element of the matrix material, there is a tendency to improve the dispersibility of the light-emitting substance in the matrix material, and to maintain the average distance between the light-emitting substance in a suitable range. The mol ratio is more preferably 0.05 or more, and even more preferably 0.1 or more. Further, the upper limit of the above mole ratio is preferably 1.5 or less, more preferably 0.2 or less, from the viewpoint of preventing segregation of only a liquid crystal phase to the outside of the particle or the particle surface of the surfactant.

In the case that the matrix material is prepared by a condensation reaction, it is preferable to use a cationic surfactant. By using a cationic surfactant, it is possible to prevent phase separation by electrostatic interaction, and a state with excellent dispersibility can be formed. Further, it is thought that if the surfactant is dispersed in advance in the solution before adding the matrix material, mycelles are formed with the hydrophilic groups facing outwards, and amorphous cluster structures of silica or titania or the like, and the light-emitting substance will agglomerate in a conjugated state on a nanoscale. In this way, there is a tendency to improve the dispersibility of the light-emitting substance in the matrix material, and maintain the average distance of the light-emitting substance in a suitable range, which is a preferable condition. Further, depending for example on the matrix material, a nonionic surfactant or an anionic surfactant may be used instead of the cationic surfactant.

The average particle diameter of the light-emitting nanoparticle is preferably 10 nm to 500 nm. By making the average particle diameter within this range, it becomes easy for the particles to be taken up by the target cells, and is also suitable in the context of observing the cells. On the other hand, if the average particle diameter is small, there is a tendency to have an effect on the active functions of the cell, and for the problem of toxicity to arise, which is not preferable. The average particle diameter of the light-emitting nanoparticle is more preferably 50 nm to 450 nm, and even more preferably 100 to 400 nm. Further, in the case that the present light-emitting nanoparticles are not used, for example, for cell imaging, the average particle diameter may be larger than 500 nm.

The light-emitting nanoparticle is preferably provided at its surface with pores with a pore diameter of 0.1 to 10 nm. By firing or solvent extracting the light-emitting nanoparticle surface, there is a tendency to form pores with a pore diameter of 0.1 to 10 nm on the light-emitting nanoparticle surface. By providing pores with a pore diameter of 0.1 to 10 nm, it is possible, for example, to support small molecule drugs on the light-emitting nanoparticle, and the use as a therapeutic agent also become possible. The pore diameter of the pores is more preferably 1 nm to 10 nm, and even more preferably 2 nm to 6 nm. Further, the light-emitting nanoparticle, depending for example on the application, may also not be provided with pores with a pore diameter of 0.1 to 10 nm on its surface. When the matrix material is silica, there is a tendency for pores to be formed. This can be thought to be because the interactions between the surfactant and the silica which is the matrix material comprising the light-emitting substance are relatively weak, and the surfactant is eliminated by the firing or solvent extraction process, whereby pores are formed. On the other hand, in the case that the matrix material is titania or a calcium phosphate compound, there is a tendency that pores are difficult to form. This is conjectured to be because the interaction between the surfactant, and the titania or calcium phosphate compound is strong, and it is difficult to eliminate the surfactant.

At the surface of the light-emitting nanoparticle, a hydroxide group (OH) bonded to the cationic element of the matrix material is preferably formed. Further, the surface of the matrix material is also preferably modified by an amino group, and for example, may also be formed using a silane coupling agent comprising an amino group. The OH group and amino group are not limited to the surface inside the pores, and may be on the particle surface such as the surface outside of the pores, and the surface outside the pores is preferable. The OH group or amino group is fixed by covalent bonding by hydrogen bonding or condensation polymerization to the cell bonding molecule, and if the surface of the light-emitting nanoparticle is modified by the cell bonding molecule, the cell bonding molecule can selectively bond to a cancer cell or a normal cell. If the cell bonding molecule selectively binds to a cell, the light-emitting nanoparticle is taken up into the cell. In this way, the light-emitting nanoparticle is made to emit light inside the cell, and it becomes possible to detect cancer or the like inside a cell. Further, according to the application of the light-emitting nanoparticle, the OH group and amino group do not have to be formed at the surface.

As the cell bonding molecule, HER2 antibody, antibodies selectively binding to human epidermal growth factor receptor, cancer-specific antibodies, phosphorylation protein antibodies, folic acid, antibodies selectively binding to folic acid receptor β, vascular endothelial cell-specific antibodies, tissue-specific antibodies, transferrin, transferring-bonding peptide, proteins having affinity to sugar chains, and the like may be mentioned. Among these, folic acid, which has tendency to be taken up by cancer cells, is preferably used as a cell bonding molecule. Because folic acid receptors are over-expressed on cell membranes for cancer cells, there is a tendency for folic acid molecules to be selectively bonded and taken up.

Further, the surface of the light-emitting nanoparticle may be modified with an anticancer agent molecule. If the anticancer agent molecule selectively binds to the cancer cell, the light-emitting nanoparticle will be taken up into the cell. In this way, the light-emitting nanoparticle is made to emit light inside the cell, and the cancer cells can be detected, and further, the anticancer agent is also taken up by the cell, and the anticancer agent molecule can operate, and can suppress the proliferation of cancer cells. Further, the light-emitting nanoparticle of the present embodiment has a wide range of applications other than cancer cells, and therefore the surface does not have to be modified with an anticancer agent molecule.

The cell bonding molecule or anticancer agent molecule are preferably modified and fixed to the surface of the light-emitting nanoparticle by a chemical bond. As the chemical bond, a peptide bond (-CO-NH-), hydrogen bond or the like may be mentioned.

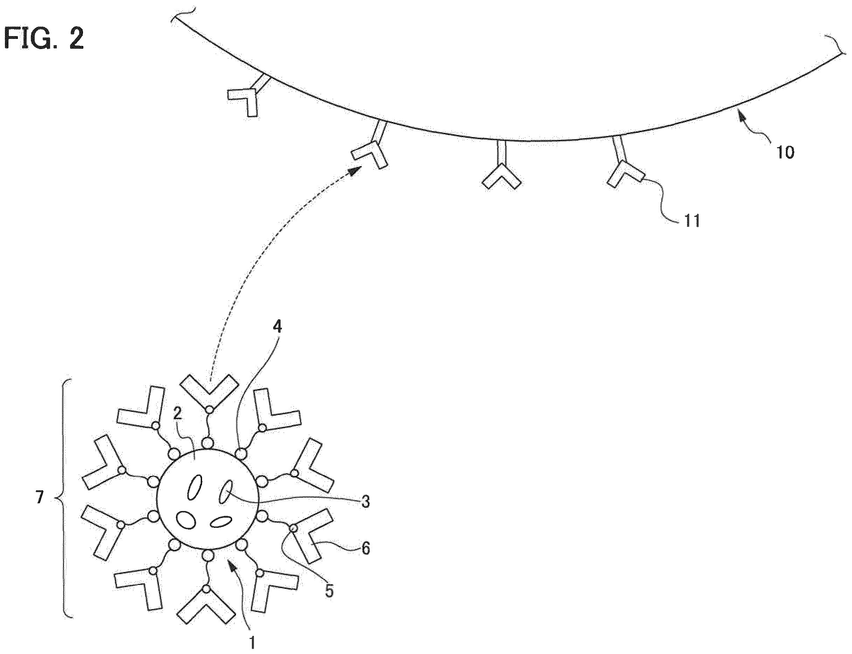

Fig. 2 is a diagram schematically showing the mechanism by which the light-emitting nanoparticle which one embodiment of the present invention is taken up in a cancer cell. As shown in Fig. 2, the light-emitting nanoparticle 1 provided with the matrix material 2 comprising the light-emitting substance 3, may form a cell binding molecule-modified light-emitting nanoparticle 7 having a bonding means 4 of an OH group or an amino group at the surface of the matrix material 2, which is bonded to a cell bonding molecule 6 by a peptide bond 5. The cell bonding molecule-modified light-emitting nanoparticle may bond to a receptor 11 of the cancer cell 10, and be taken up into the cancer cell 10.

For the light-emitting nanoparticles of the present embodiment, the excitation wavelength and the light-emission wavelength are preferably in the visible light region. If the excitation wavelength and the light-emission wavelength are in the visible light wavelength or higher, degradation of biological tissue and labeling material can be reduced. Further, optical scattering of the test surface can be reduced, and the observation sensitivity can be increased. Moreover, in an application using the light-emitting nanoparticles, in cases where it is not necessary to consider damage to biological tissue and labeling material, the excitation wavelength and the light-emission wavelength are preferably do not have to be in the visible light region.

The light-emitting nanoparticles of the present embodiment are preferably used for biological imaging. In the case that an organic molecule is used as the matrix material, the degradation and fading speed are fast, and there has been the problem that by exciting with ultraviolet rays, normal biological tissue is damaged. Further, in the case of using an inorganic material such as a quantum dot or the like, there are problems of biocompatibility such as the inclusion of highly toxic elements and the like, and the wavelengths of the excitation light include the ultraviolet region, and therefore, there has been concern of imparting damage to biological tissue. In contrast, the light-emitting nanoparticle of the present embodiment is provided with light-emitting stability and light resistance, and damage to biological tissue can be reduced, and living body toxicity is also low, whereby it can be suitably applied to biological imaging. Further, the light-emitting nanoparticle of the present embodiment is in a state which is preferable for use in applications other than biological imaging.

EXAMPLES

Below, specific examples of the present invention are explained. Further, the present invention is not to be interpreted as being limited by these examples.

<Examples 1, 2, Comparative Example 1>

(Synthesis of $Eu^{3+}$-containing silica particles)

To 225 mL of deionized water, 1.0 g of cetyltrimethylammonium bromide (CTAB) was added, and further, 3.5 mL of 2.0 M NaOH was added, and stirring was carried out at 353 K for 30 min. To the stirred solution, 5.515 mL of tetraethoxysilane (TEOS), and 15 mL of deionized water containing $EuCl_3$ were added (when the $EuCl_3$ was 0 g, the charge-in quantity at the synthesis starting time of Eu was 0 mol%, and is shown as "Eu0mol%-S" (Comparative Example 1). When the $EuCl_3$ was 0.452 g, the charge-in quantity at the synthesis starting time of Eu was 5 mol%, and is shown as "Eu5mol%-S" (Example 1). When the $EuCl_3$ was 0.904 g, the charge-in quantity at the synthesis starting time of Eu was 10 mol%, and is shown as "Eu10mol%-S" (Example 2)), stirring was carried out at 353 K for 2 hours, and filtered. The residue was washed 4 times with 20 mL deionized water, and 1 time with 10 mL ethanol. After this, it was dried for 1 day at room temperature, and fired at 550°C for 6 hours. The concentrations and the like of the elements constituting the particles of Examples 1 and 2, and Comparative Example 1 are as shown in Table 1.

[Table 1]

| Sample name | Constituent element concentration (mol%) | | | | | | | | Mol ratio of light-emitting substance with respect to matrix element |
|---|---|---|---|---|---|---|---|---|---|
| | Si | O | Na | P | Ti | K | Ca | Eu | Eu/Si |
| Example 1 Eu5mol%-S | 22.8 | 76.1 | 0.00 | 0.00 | 0.00 | 0.00 | 0.00 | 1.06 | 4.65 |
| Example 2 Eu10mol%-S | 27.4 | 69.9 | 0.00 | 0.00 | 0.00 | 0.00 | 0.00 | 2.68 | 9.78 |
| Comparative Example 1 Eu0mol%-S | 23.1 | 76.9 | 0.00 | 0.00 | 0.00 | 0.00 | 0.00 | 0.00 | 0.00 |

<Example 3, Comparative Examples 2, 3>

(Synthesis of fluorescein isothiocyanate (FITC)-containing titania particles)

To 0.011 mL ($4.63 \times 10^{-5}$ mol) of 3-aminopropyltriethoxysilane (APTES: $C_9H_{23}NO_3Si$), 0 mg (0 mol, Comparative Example 2), or 91.1 mg (Example 3), or 192 mg ($4.68 \times 10^{-4}$ mol, Comparative Example 3) of fluorescein isothiocyanate (FITC: $C_{21}H_{11}NO_5S$), and 36.1 mL (0.471 mol) of 2-propanol (IPA) were mixed, and stirring was carried out for 24 hours using a magnetic stirrer. To this solution, 1.37 mL ($4.68 \times 10^{-3}$ mol) of titanium tetraisopropoxide (TTIP: $C_{12}H_{28}O_4Ti$) was added so that the Ti/APTES mol ratio became = 100, whereby the solution A was prepared. The charge-in quantity at the synthesis starting time of the FITC of Comparative Example 2 was shown as 0 mol%, "FITC0mol%-T", the charge-in quantity at the synthesis starting time of the FITC of Example 3 was 5 mol%, "FITC5mol%-T", and the charge-in quantity at the synthesis starting time of the FITC of Comparative Example 3 was 10 mol%, "FITC10mol%-T". 37.3 mL (0.487 mol) of IPA and 0.231 mL ($1.28 \times 10^{-2}$ mol) of ion-exchanged water were mixed, whereby solution B was prepared. 205 mg ($7.61 \times 10^{-4}$ mol) of octadecylamine (ODA: $C_{18}H_{39}N$), 189 mL (2.47 mol) of IPA, and 0.900 mL ($4.99 \times 10^{-2}$ mol) of ion-exchanged water were mixed, whereby solution C was prepared in a polypropylene vessel. Herein, APTES was expected to express an interaction accompanying the formation of hydrogen bonds or the like of ODA and FITC. The solutions A and B were liquid-fed at respective flow rates of 30 mL·min$^{-1}$ and mixed. Using IPA as the good solvent for TTIP, APTES, FITC and ODA, and using ion exchanged water as the reactant for TTIP and APTES hydrolysis, ODA was used for control of the form, size and nanostructure of the product. The reaction liquid thereof was delivered at a flow rate of 60 mL·min$^{-1}$ to the vessel of the solution C, and until the end of delivery, stirring was carried out with a magnetic stirrer, and after this, was allowed to stand at room temperature for 24 hours, and a particle dispersion was obtained. Solid-liquid separation was done by centrifuge (9000 rpm, 10 min), and after discarding the supernatant liquid, the precipitate was dried overnight at 60°C, and a sample powder was obtained. The concentrations and the like of the elements constituting the particles of Example 3, and Comparative Examples 2 and 3 are as in Table 2.

[Table 2]

| Sample name | Constituent element concentration (mol%) | | | | | | | | | Mol ratio with respect to matrix elements |
|---|---|---|---|---|---|---|---|---|---|---|
| | Si | O | Na | P | Ti | K | Ca | C | S | FITC/Ti |
| Example 3 FITC5mol%-T | 0.00 | 38.5 | 0.00 | 0.00 | 15.1 | 0.00 | 0.00 | 45.8 | 0.60 | 4.0 |
| Comparative Example 3 FITC10mol%-T | 0.00 | 38.3 | 0.00 | 0.00 | 14.9 | 0.00 | 0.00 | 45.4 | 1.42 | 9.5 |
| Comparative Example 2 FITC0mol%-T | 0.00 | 38.4 | 0.00 | 0.00 | 17.4 | 0.00 | 0.00 | 44.1 | 0.0 | 0.0 |

<Examples 4, 5, Comparative Example 4>

(Synthesis of $Eu^{3+}$-containing calcium phosphate compound particles)

To a solution consisting of 100 mL $H_2O$ (80°C), and 8.75 g cetyltrimethylammonium bromide (CTAB, molecular weight 364.45) (0.024 mol), 2.09 g (0.012 mol) $K_2HPO_4$, and 1N NaOH were added by dropwise, so that the solution pH became 13, and cooled to 40°C or less. Next, 60 mL $H_2O$, $CaCl_2 \cdot 2H_2O$ was 2.87 g (0.0195 mol), and $EuCl_3 \cdot 6H_2O$ was 0g (0 mmol), 0.357 g (0.9 mmol), or 0.714 g (1.9 mmol). The charge-in quantity at the synthesis starting time of the Eu of Comparative Example 4 was shown as 0 mol%, "Eu0mol%-CP", the charge-in quantity at the synthesis starting time of the Eu of Example 4 was 5 mol%, "Eu5mol%-CP", and the charge-in quantity at the synthesis starting time of the Eu of Example 5 was 10 mol%, "Eu10mol%-CP". These Eu-containing solutions were added dropwise to the solution cooled to 40°C or less, at a dropping flow rate of 6 mL/min. After the dropping, while stirring, this was reflux heated for 24 hours at 40°C. The obtained white precipitate was washed two times with pure water, and washed two times with ethanol. After washing, and centrifuging (10000 G, 15 min, 4°C), drying was carried out for 24 hours at 100°C. The concentrations and the like of the elements constituting Examples 4 and 5, and Comparative Example 4, as as in Table 3.

[Table 3]

| Sample name | Constituent element concentration (mol%) | | | | | | | | Mol ratio with respect to matrix elements |
|---|---|---|---|---|---|---|---|---|---|
| | Si | O | Na | P | Ti | K | Ca | Eu | Eu/Ca |
| Example 4 Eu5mol%-CP | 0.00 | 60.8 | 0.10 | 10.4 | 0.00 | 0.01 | 20.1 | 0.75 | 3.73 |
| Example 5 Eu10mol%-CP | 0.00 | 61.0 | 0.17 | 11.1 | 0.00 | 0.02 | 21.2 | 1.77 | 8.34 |
| Comparative Example 4 Eu0mol%-CP | 0.00 | 59.2 | 0.17 | 10.8 | 0.00 | 0.02 | 19.6 | 0.00 | 0.00 |

(Observation of the light-emitting nanoparticles by TEM)

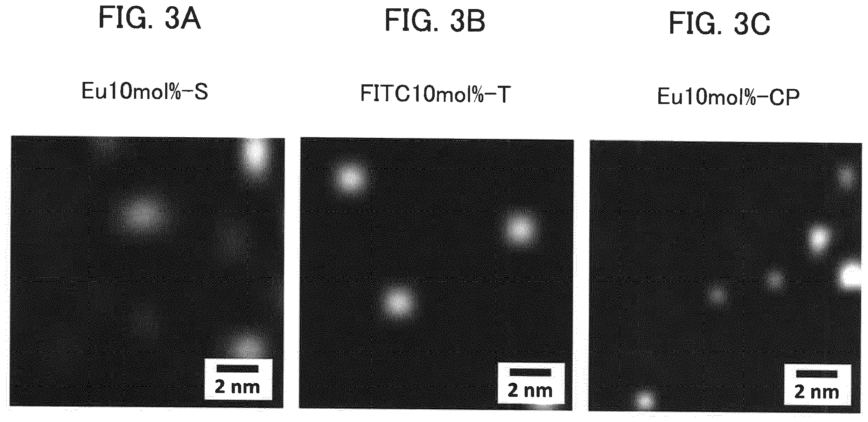

For the light-emitting nanoparticles of Examples 2 and 5, and Comparative Example 3, observation was carried out for a light-emitting substance distribution by a transmission electron microscope (TEM). Specifically, each type of particle powder was dispersed at a concentration of 1 wt% in ethanol, an ultrasound wave treatment was applied for 15 min, and the particle suspension liquid was cast on a glass substrate with a concentration of 0.01 mL/cm$^2$. Vacuum drying was applied for 1 day, carbon deposition (film thickness: 10 nm) was applied to the substrate surface, a cross section of the particle film was cut out with a focused ion beam (surface area 8 $\mu$m $\times$ 6 pm), and mounted on a carbon micro grid. Next, the central portion of the particle film was evaluated and analyzed by the transmission electron microscope (TEM) (HT 7700 by Hitachi High Technologies K.K.), and attached EDS (energy dispersion type X-ray spectroscopy). The observation results are shown in Fig. 3. In Fig. 3, the light-emitting substance is present as single molecules or ions with a white, approximately circular shape, and it could be confirmed that they are present distributed inside the light-emitting nanoparticles.

(Measurement method of the average distance between the light-emitting substance)

It could be confirmed by TEM that the light-emitting substance was distributed in the matrix material, and therefore, the average distance between the light-emitting substance was calculated from the average particle diameter and concentration of the light-emitting substance. Specifically, the distance between the light-emitting substance was calculated by a density computation of the light-emitting substance with respect to the metal element of the inorganic phase which is the matrix material by fluorescence X- ray (XRF) analysis and scanning electron microscope (FE-SEM) observation.

(1) Calculation of the inorganic molecule number density of the inorganic phase

As shown below in Table 4, by the density (known value) of the inorganic phase, the molecular number density of the inorganic phase was calculated.

[Table 4]

| Inorganic phase type | cm density (g/cm$^3$) (Notes 1 to 3) | nm density (g/cm$^3$) | Inorganic molecular mol density (mol/nm$^3$) | Inorganic molecular number density (molecule number/nm$^3$) |
|---|---|---|---|---|
| Silica phase | 1.5 | $1.5 \times 10^{-21}$ | $1.9 \times 10^{-23}$ | 11.3 |
| Titania phase | 3.0 | $3.0 \times 10^{-21}$ | $5.0 \times 10^{-23}$ | 30.1 |
| Hydroxyapatite phase | 3.2 | $3.2 \times 10^{-21}$ | $3.2 \times 10^{-24}$ | 1.9 |

(Note 1) for the density of the silica phase, a porous silica with a density of 1.50 was used (reference documents: Kazunori Iwamoto, Manabu Senoo, "Functionalization of Inorganic-Organic Compound Material", Seisan Kenkyu, 42 (8), 1990, and the like).

(Note 2) for the titania phase density, it is understood to be an amorphous phase by the XRD pattern (if not amorphous, it is difficult to include various light-emitting substances). Thus, amorphous phase titania with a density of 3.0 g/cm$^3$ was used (reference documents: M. Laube, F. Rauch, C. Ottermann, O. Anderson, and K. Bange, Nucl. Instrum. Methods Phys. Res., Sect. B, 1996, 113, 288-292; C. R. Ottermannn and K. Bange, Thin Solid Films, 1996, 286, 32-34; D. Mergel, D. Buschendorf, S. Eggert, R. Grammes and B. Samset, Thin Solid Films, 2000, 371, 218-224; D Mergel, Thin Solid Films, 2001, 397, 216-222; V. V. Hoang, H. Zung, and N. H. B. Trong, Eur. Phys. J. D, 2007, 44, 515-524, and the like).

(Note 3) for the density of hydroxyapatite (CP), because a crystalline phase of hydroxyapatite was confirmed by the XRD pattern, the density (3.2 g/cm$^3$) of crystalline phase hydroxyapatite was used.

(2) Calculation of the average particle diameter, inorganic molecule number per 1 particle

For the light-emitting nanoparticles of Examples 1 to 5, and Comparative Example 3, the particle diameters of 100 or more light-emitting nanoparticles was measured using FE-SEM, and the average particle diameter was calculated. Further, the inorganic molecule number of the inorganic phase included per 1 molecule was calculated (refer to Table 5).

[Table 5]

| Sample name | Average particle diameter: R (mm) (Note 4) | Average 1 particle volume (nm$^3$/1 particle) | Contained inorganic molecule number (molecule number/1 particle) (Note 5) |
|---|---|---|---|
| Eu5mol% -S | 346 | $2.2 \times 10^7$ | $2.5 \times 10^8$ |
| Eu10mol% -S | 286 | $1.2 \times 10^7$ | $1.4 \times 10^8$ |
| FITC5mol% -T | 418 | $3.8 \times 10^7$ | $1.1 \times 10^9$ |
| FITC10mol% -T | 423 | $4.0 \times 10^7$ | $1.2 \times 10^9$ |
| Eu5mol% -CP | 48 | $5.8 \times 10^4$ | $1.1 \times 10^5$ |
| Eu10mol% -CP | 41 | $3.6 \times 10^4$ | $6.9 \times 10^4$ |

(Note 4) because each particle has an anisotropic shape, the above average particle diameter R was calculated as {(long diameter + short diameter)/2}.

(Note 5) calculated considering the volume of the light-emitting substance to be a point (zero).

In the case of a silica phase, the correspondence of one Si per one inorganic silica molecule unit, and in the case of a titania phase, the correspondence of one Ti per one inorganic silica molecule unit, and in the case of a hydroxyapatite phase the correspondence of six Ca per one inorganic silica molecule unit was applied.

(3) Calculation of the distance between light-emitting substance

From the density of the light-emitting substance corresponding to the inorganic metal element obtained by XRF, the distance between light-emitting substance was calculated. As shown in Table 6, for the light-emitting nanoparticle produced in Examples 1 to 5 and Comparative Example 3, the average distance between the light-emitting substance included in the matrix material was 1.2 nm or more except for FITC10mol%-T, which is Comparative Example 3.

[Table 6]

| Sample name | Light-emitting substance number with respect to inorganic molecule number (molecule number/ molecule number) | Contained light-emitting substance number (molecule number/ 1 particle) | Occupied volume of light-emitting substance 1 molecule ($nm^3$/light-emitting substance 1 molecule) | Distance between light-emitting substance (nm) (Note 6) |
|---|---|---|---|---|
| Eu5mol% -S | $5.0 \times 10^{-2}$ | $1.1 \times 10^7$ | 1.9 | 1.5 |
| Eu10mol% -S | $10.0 \times 10^{-2}$ | $1.4 \times 10^7$ | 0.9 | 1.2 |
| FITC5mol% -T | $4.0 \times 10^{-2}$ | $4.6 \times 10^7$ | 0.8 | 1.2 |
| FITC10mol% -T | $9.0 \times 10^{-2}$ | $1.1 \times 10^8$ | 0.4 | 0.9 |
| Eu5mol% -CP | $4.0 \times 10^{-2}$ | $2.5 \times 10^4$ | 2.3 | 1.6 |
| Eu10mol% -CP | $8.0 \times 10^{-2}$ | $3.5 \times 10^4$ | 1.0 | 1.3 |
| (Note 6) it is assumed that the light-emitting substance is approximately uniformly individually distributed by a surfactant. | | | | |

(B) Testing of the fluorescence life

By fluorescence life measurement, the dispersibility of the light-emitting substance was tested. The sample of the light-emitting substance, in addition of the 5 mol%, 10mol% of the light-emitting substance synthesized when producing the below Examples, a sample of 2.5 mol% was also prepared. For the Eu light-emitting substance, an FP-8500 fluorospectrophotometer made by JASCO Corp. was used. For the FITC light-emitting substance, a DeltaPro fluorescence life photometer made by Horiba, Ltd. was used. For a light source, a xenon flash tube was used, for the excitation wavelength, the same wavelength as the fluorescence spectra was used, and as the test wavelength, the largest wavelength of the fluorescence spectra was used. The slit bandwidth at the excitation side and the receiving side was 2 nm. From immediately after the lighting of the flash lamp, for the light-emitting substance Eu, the florescence intensity changes were measured for an interval of 50 ms, and the light-emitting substance FITC, the florescence intensity changes were measured for an interval of 200 ns, and these decay curves of fluorescence intensity were measured with 10 repetitions. These 10 batches of decay curves were fit to the below Formula (7), and the fluorescence life $\tau$ was calculated.

$$I(t) = I(0)\exp(-t/\tau) \quad \text{Formula (7)}$$

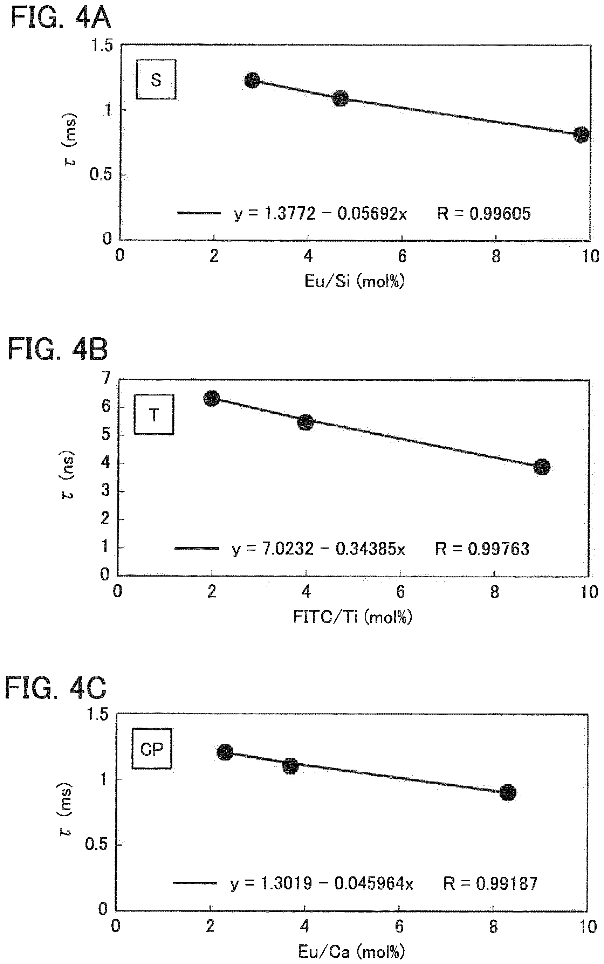

Herein, I(t) is the fluorescence intensity at the time t, I(0) is the fluorescence intensity immediately after the lighting of the flash lamp. As a result, the fluorescence life $\tau$ was as shown below in Table 7. Then a plot with the light-emitting substance density on the horizontal axis, and the fluorescence life $\tau$ on the vertical axis was prepared. The results are shown in Fig. 4.

[Table 7]

| Sample name | Light-emitting substance content concentration (mol%) | Fluorescence life |
|---|---|---|
| Eu2.5mol%-S | 2.78 | 1.2 |
| Eu5mol%-S | 4.65 | 1.1 |

(continued)

| Sample name | Light-emitting substance content concentration (mol%) | Fluorescence life |
|---|---|---|
| Eu10mol%-S | 9.78 | 0.8 |
| FITC2.5mol%-T | 2.00 | $6.4 \times 10^6$ |
| FITC5mol%-T | 4.00 | $5.6 \times 10^6$ |
| FITC10mol%-T | 9.00 | $4.0 \times 10^6$ |
| Eu2.5mol%-CP | 2.32 | 1.2 |
| Eu5mol%-CP | 3.73 | 1.1 |
| Eu10mol%-CP | 8.34 | 0.9 |

Fig. 4(a) shows the relationship between the light-emitting substance concentration and the fluorescence life for a silica phase (S), 4(b) for a titania phase (T), and 4(c) for hydroxyapatite (CP), and as shown in Fig. 4(a) to (c), the plot of fluorescence life with respect to each concentration shows a negative linear correlation. This correlation is a monotonic decrease, therefore, it is surmised that this is an equivalent matrix environment with respect to the light-emitting substance. Namely, along with a concentration increase of the light-emitting substance, the occupied volume contributed to the light-emitting substance is linearly reduced, the distance between the light-emitting substance becomes short, and the probability of cross relaxation process becomes high (the excitation energy is partially transferred to proximal ions, and the resulting two low excitation state ions display the phenomenon of rapid relaxation to the base state). The correlation between the light-emitting substance concentration and fluorescence life shows a high correlation of 0.95 or more, and it can be considered that the light-emitting substance itself is present approximately uniformly distributed as individual molecules or ions which do not relax each other, and the distance between the light-emitting substance is small. As above, it was confirmed that in the light-emitting nanoparticles used in the Examples, the light-emitting substance was present approximately uniformly distributed.

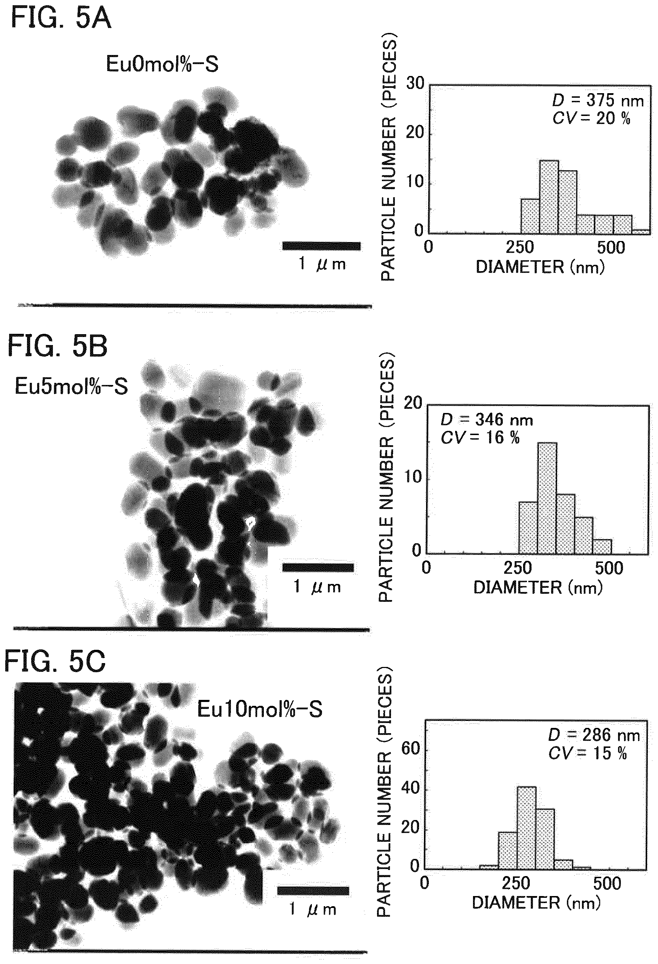

Fig. 5 is an electron microscope observed image (TEM image) by concentration of $Eu^{3+}$-containing silica particles. Fig. 5(a) is Comparative Example 1 (Eu0mol%-S), Fig. 5(b) is Example 1 (Eu5mol%-S), and Fig. 5(c) is Example 2 (Eu10mol%-S). Figs. 5(a) to (c), show the trend that as the concentration of Eu becomes higher, the average particle diameter (D) of the $Eu^{3+}$-containing silica particles becomes smaller. Further, the coefficient of variation (CV), which is a relative standard deviation, was 15 to 20%.

The aspect ratio of the particles of Examples 1 and 2 and Comparative Example 1 were as shown below in Table 8. The aspect ratio is determined by dividing the long axis size of the particle by the short axis size. As the concentration of Eu becomes higher, the aspect ratio is reduced, and the particles show a change in shape from needle shape to spherical shape.

[Table 8]

| Sample name | Aspect ratio of the particle |
|---|---|
| Example 1 (Eu5mol%-S) | $1.17 \pm 0.11$ |
| Example 2 (Eu10mol%-S) | $1.07 \pm 0.11$ |
| Comparative Example 1 (Eu0mol%-S) | $1.23 \pm 0.18$ |

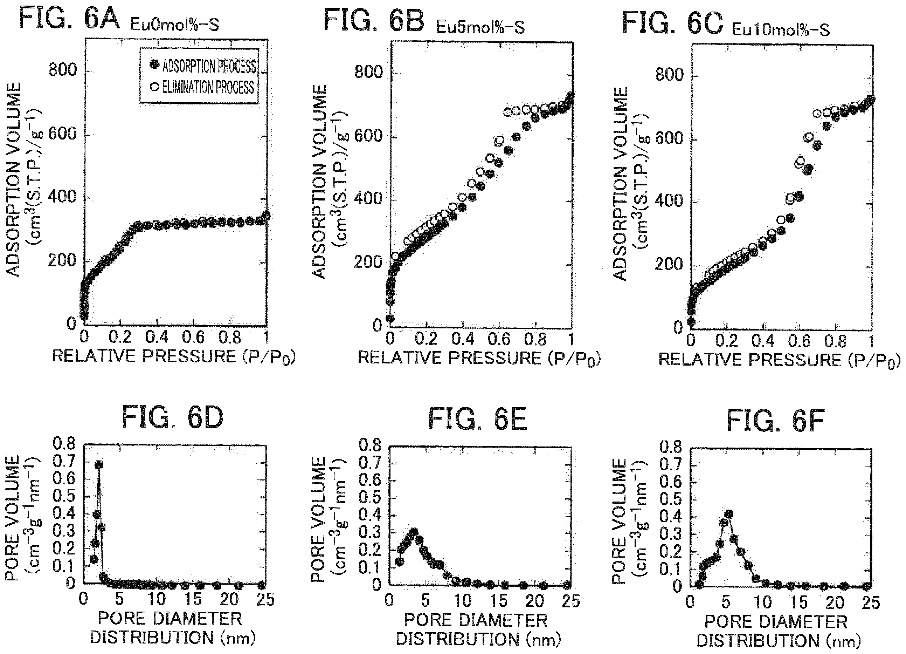

The average distance between the light-emitting substance in the $Eu^{3+}$-containing silica particles of Examples 1 and 2 were respectively 1.5 nm and 1.2 nm (refer to the above Table 6). Further, concerning the $Eu^{3+}$-containing silica particles of Examples 1 and 2, by solvent extraction or firing (oxidative decomposition) of the surfactant, pores with diameters in a range of 1 to 10 nm were observed. The analysis results of the specific surface area and pore diameter are shown in Table 9. Further, Fig. 6 shows the nitrogen absorption/desorption isotherm and pore diameter distribution. Figs. 6(a) and (d) relate to Eu0mol%-S, 6(b) and (e) relate to Eu5mol%-S, and Figs. 6(c) and (f) relate to Eu10mol%-S. Concerning the measurement method, the BET specific surface area and (determined by the BJH method) BJH pore diameter distribution were measured by the nitrogen absorption/desorption isotherm (BELSORP-mini manufactured by MicrotracBEL Corp.). The sample was degassed for a whole day and night, dried for 12 hours at 100°C, and measured at an adsorption temperature of -196°C and maximum equilibrium pressure of 760 Torr. As a result, as shown in Table 9, it was confirmed that the mesopores were enlarged along with an increase in the doping amount of Eu. The distribution center of the included mesopore diameter was approximately 2 to 6 nm.

[Table 9]

| Sample name | BET specific surface area (m²/g) | Center mesopore diameter (nm) |
|---|---|---|
| Eu0mol%-S | 1079 | 2.2 |
| Eu5mol%-S | 1039 | 3.7 |
| Eu10mol%-S | 741 | 5.4 |

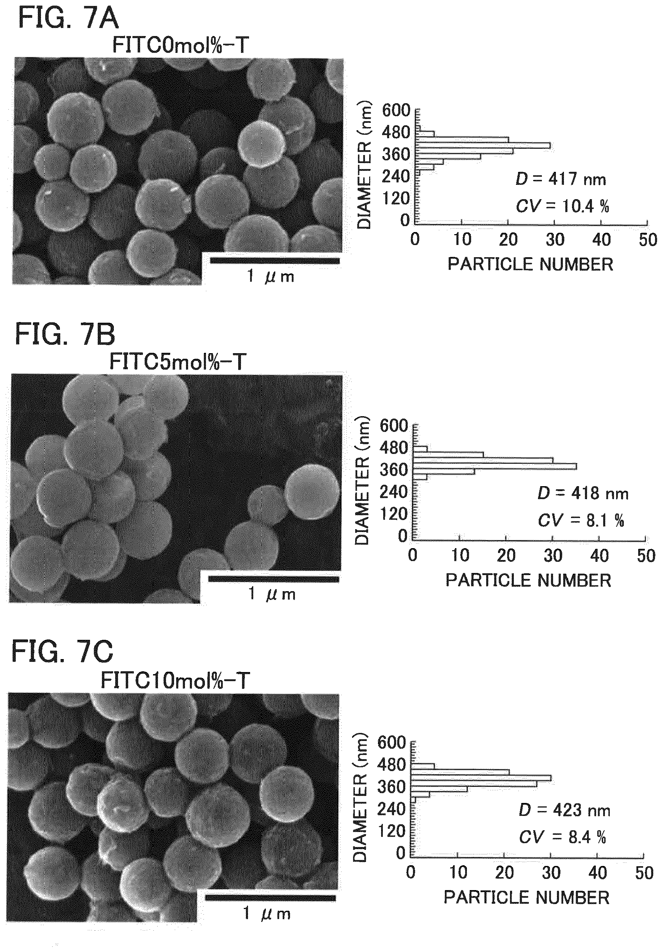

Fig. 7 is an electron microscope observed image (FE-SEM) by density of the FITC-containing titania particles, and a particle diameter distribution. Fig. 7(a) is Comparative Example 2 (FITC0mol%-T), Fig. 7(b) is Example 3 (FITC5mol%-T), and Fig. 7(c) is Comparative Example 3 (FITC10mol%-T). Figs. 7(a) to (c) show a tendency that as the density of the FITC becomes higher, the average particle diameter (D) of the FITC-containing titania particles becomes greater. Further, the coefficient of variation (CV) was 8.1 to 10.4%.

The aspect ratios of the particles of Example 3 and Comparative Examples 2 and 3 were as shown in the following Table 10. The aspect ratio was determined by dividing the long axis size of the particle by the short axis size.

[Table 10]

| Sample name | Aspect ratio of the particle |
|---|---|
| Example 3 (FITC5mol%-T) | 1.01±0.01 |
| Comparative Example 3 (FITC10mol%-T) | 1.00±0.01 |
| Comparative Example 2 (FITC0mol%-T) | 1.01±0.01 |

The average distance between the light-emitting substance in the FITC-containing titania particles of Example 3 and Comparative Example 3 were respectively 1.2 nm and 0.9 nm (refer to Table 6), and showed a tendency to decrease along with a density increase of the light-emitting substance. Further, concerning the FITC-containing titania particles of Example 3, by solvent extraction or firing (oxidative decomposition) of the surfactant, pores were observed. This is conjectured to be because the interaction between the surfactant and the FITC-containing titania particles of Example 3 is stronger than the interaction between the surfactant and the $Eu^{3+}$-containing silica particles of Examples 1 and 2, whereby it was difficult to eliminate the surfactant.

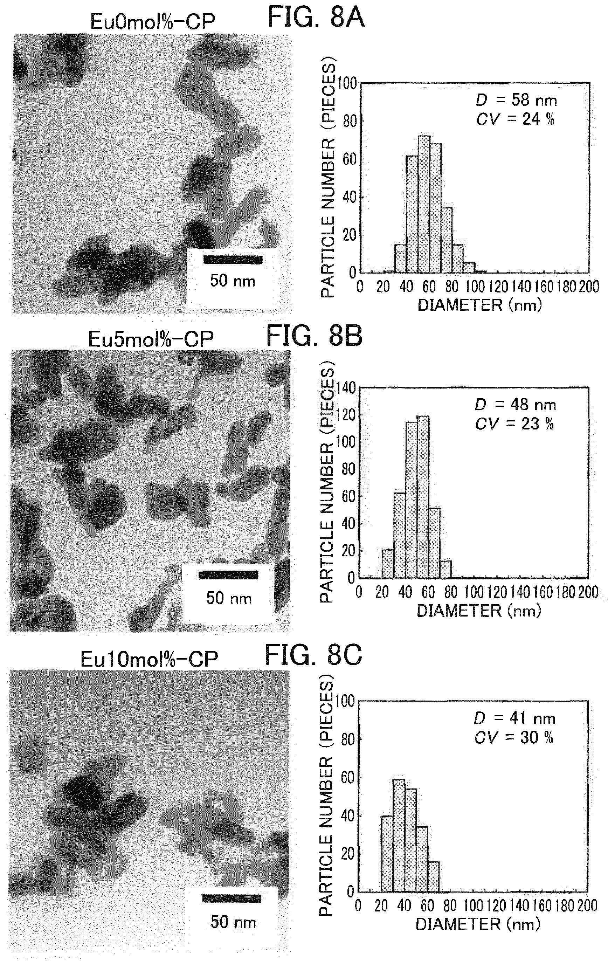

Fig. 8 is an electron microscope observed image (TEM image) by concentration of $Eu^{3+}$-containing calcium phosphate compound particles. Fig. 8(a) is Comparative Example 4 (Eu0mol%-CP), Fig. 8(b) is Example 4 (Eu5mol%-CP), and Fig. 8(c) is Example 4 (Eu10mol%-CP), Figs. 8(a) to (c) show a trend that the trend that as the concentration of Eu becomes higher, the average particle diameter (D) of the $Eu^{3+}$-containing calcium phosphate compound particles becomes smaller. Further, the coefficient of variation (CV) was 23 to 30%.

For the aspect ratios of the particles of Examples 4 and 5, and Comparative Example 4, Table 11 below shows that, as the concentration of Eu increases, the aspect ratio decreases, and the particles change from a needle shape to a spherical form.

[Table 11]

| Sample name | Aspect ratio of the particle |
|---|---|
| Example 4 (Eu5mol%-CP) | 1.89±0.13 |
| Example 5 (Eu10mol%-CP) | 1.53±0.18 |
| Comparative Example 4 (Eu0mol%-CP) | 2.43±0.12 |

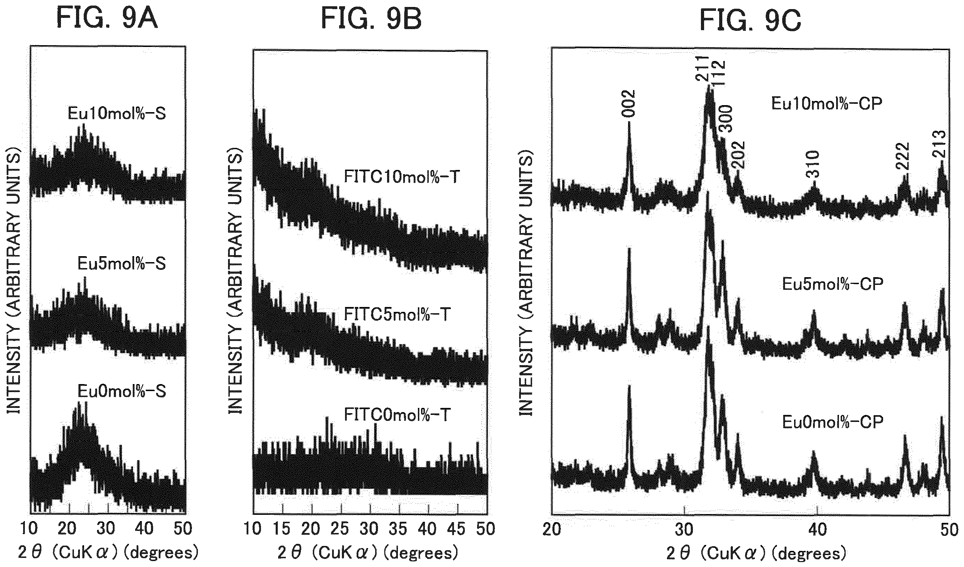

Fig. 9 is a graph showing X-ray diffraction patterns, (a) $Eu^{3+}$-containing silica particles, (b) FITC-containing titania particles, and (c) $Eu^{3+}$-containing calcium phosphate compound particles. Fig. 9(a) shows an amorphous structure, wherein the peaks deriving from the precipitate of Eu and the silica crystals were not observed, and shows a tendency wherein the greater the content of Eu, the lower the peak intensity. Fig. 9(b) shows a tendency that the greater the FITC content, the higher the peak intensity of the left end side of the graph. In Fig. 9(c), from the assignment of the Miller indices, the crystal structure is a hydroxyapatite single phase, and it is seen that as the Eu content becomes greater, portions where the peak width at half height becomes lower were seen.

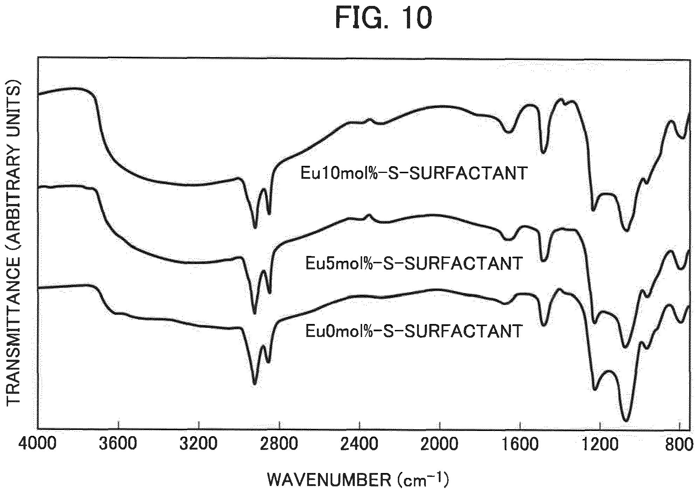

Fig. 10 is a graph showing the infrared absorption spectrum before surfactant removal for the $Eu^{3+}$-containing

silica particles. Characteristic absorption bands of an Si-OH stretching vibration of a hydrogen bond type at 3640 $cm^{-1}$, a C-H stretching vibration at 2925 $cm^{-1}$ ($-CH_3$), a C-H stretching vibration at 2855 $cm^{-1}$ ($-CH_2-$), a bending vibration at 1480 $cm^{-1}$ ($-CH_2-$), an Si-O-Si asymmetric stretching vibration at 1225 $cm^{-1}$ (($Si-O-Si$)$_n$ derived), an Si-O-Si symmetric stretching vibration at 1070 $cm^{-1}$ (($Si-O-Si$)$_n$ derived), an Si-OH stretching vibration at 965 $cm^{-1}$, and an Si-OH stretching vibration at 795 $cm^{-1}$, and the like were observed. The presence of a surfactant was confirmed by the presence of absorption bands of a C-H stretching vibration at 2925 $cm^{-1}$ ($-CH_3$), a C-H stretching vibration at 2855 $cm^{-1}$ ($-CH_2-$), and a bending vibration at 1480 $cm^{-1}$ ($-CH_2-$).

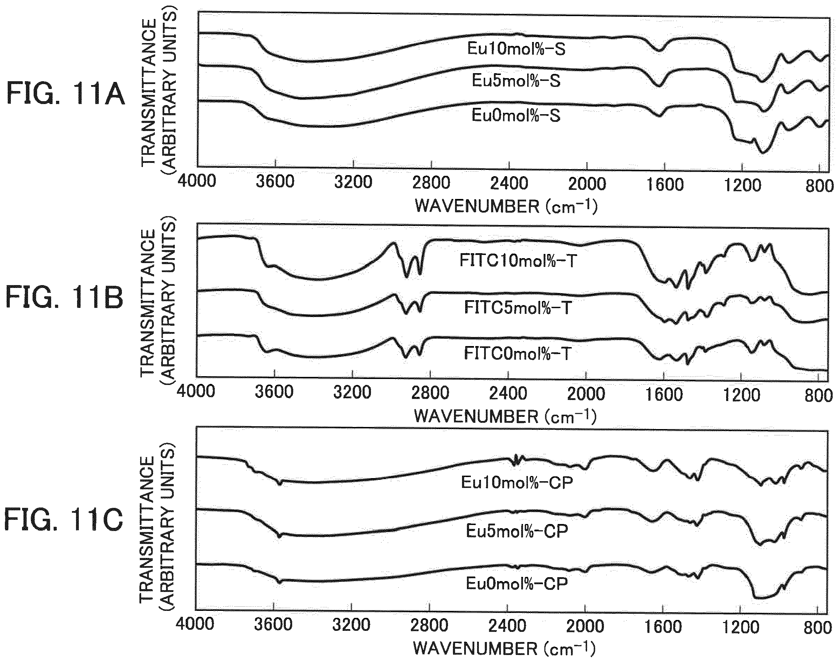

Fig. 11 is a graph showing the infrared absorption spectrum, (a) $Eu^{3+}$-containing silica particles, (b) FITC-containing titania particles, and (c) $Eu^{3+}$-containing calcium phosphate compound particles. In Fig. 11(a), characteristic absorption bands of an Si-OH stretching vibration of a hydrogen bond type at 3640 $cm^{-1}$, an Si-O-Si asymmetric stretching vibration at 1225 $cm^{-1}$ (($Si-O-Si$)$_n$ derived), an Si-O-Si symmetric stretching vibration at 1070 $cm^{-1}$ (($Si-O-Si$)$_n$ derived), an Si-OH stretching vibration at 965 $cm^{-1}$, and an Si-OH stretching vibration at 795 $cm^{-1}$, and the like were observed. As a result of the firing or solvent extracting processes, the absorption bands of a C-H stretching vibration at 2925 $cm^{-1}$ ($-CH_3$), a C-H stretching vibration at 2855 $cm^{-1}$ ($-CH_2-$), a bending vibration at 1480 $cm^{-1}$ ($-CH_2-$) disappear, whereby it was judged that the surfactant was eliminated. In Fig. 11(b) characteristic absorption bands of a stretching vibration of an OH group in a titania structure at 3640 $cm^{-1}$, a stretching vibration of a Ti-OH and $H_2O$ of a particle surface at 3720-3000 $cm^{-1}$, a stretching vibration of a $-CH_3$ and $-CH_2-$ caused by surfactant ODA (octadecylamine) and the light-emitting substance FITC at 2920 $cm^{-1}$ and 2850 $cm^{-1}$, a bending vibration of $-CH_2-$ at 1460 $cm^{-1}$, and a stretching vibration of C=O at 1590 $cm^{-1}$ were observed. Ultimately, the surfactant survived the washing process by IPA. From this, it was surmised that the surfactant survived due to the interaction between the surfactant and titania/FITC. In Fig. 11(c) characteristic absorption bands of a stretching vibration of an OH group in a crystal structure of hydroxyapatite at 3550 $cm^{-1}$, P-O stretching vibrations of phosphate groups at 1100 $cm^{-1}$, 1000 $cm^{-1}$, and 960 $cm^{-1}$, stretching vibrations of OH groups of $H_2O$ of a particle surface at 3800-3000 $cm^{-1}$ and 1650 $cm^{-1}$, and the like were observed. The P-O and -OH stretching vibrations which are characteristic peaks of calcium phosphate (especially, hydroxyapatite) were observed. Ultimately, the surfactant was not observed. This is because the surfactant was sufficiently eliminated by the washing. It was confirmed by the XRF results that the surfactant was ultimately eliminated by the washing process, and pores were formed for CP.

The average distances between the light-emitting substance in the $Eu^{3+}$-containing calcium phosphate compound particles of Examples 4 and 5 were respectively 1.6 nm and 1.3 nm (refer to Table 6). For the $Eu^{3+}$-containing calcium phosphate compound particles of Examples 4 and 5, pores were not observed due to the solvent extracting processes or firing (oxidative decomposition) of the surfactant. This is surmised to be because the interaction between the surfactant and the $Eu^{3+}$-containing calcium phosphate compound particles is stronger than the interaction between the surfactant and the $Eu^{3+}$-containing silica particles of Examples 1 and 2, whereby surfactant in the $Eu^{3+}$-containing calcium phosphate compound particles was difficult to eliminate.

<Examples 6 to 8>

(Modification of cancer cell binding molecule (folic acid derivative FA-NHS) with light-emitting substance 5 mol%-containing particles)

To 250 mg of the particles containing 5 mol% of each light-emitting substance of Examples 1, 3, and 4, 12 ml of an HCl aqueous solution (pH = 2) was added, and treatment with ultrasonic waves was carried out. Next, a solution containing 0.78 ml (3.3 mmol) of 3-aminopropyltriethoxysilane (APTES) in 5 mL of ethanol was prepared, and added to the ultrasonic wave-treated solution, and a mixed solution was obtained. This mixed solution was stirred for 20 hours at 40°c (pH < 6.5). After completion of the stirring, the mixed solution was separated by centrifuge, and washed with ethanol. After washing, it was vacuum dried, and 150 mg of particles containing 5 mol% light-emitting substance with a surface modified by APTES was obtained. To 150 mg of these APTES/light-emitting substance 5 mol%-containing particles, 25 mL of a 50 mM phosphate buffer solution (pH = 7.0) was added, and treatment with ultrasonic waves was carried out. Next, a solution comprising 430 mg (0.8 mmol) of FA-NHS in 12 mL of dimethylsulfoxide (DMSO) was prepared, and added to the ultrasonic wave-treated solution, and a mixed solution was obtained. This mixed solution was stirred for 3 hours at room temperature. After completion of the stirring, the mixed solution was separated by centrifuge, and washed with water. After washing, it was vacuum dried, and the particles containing 5 FA (folic acid)/light-emitting substance 5 mol% of Examples 6 to 8 were obtained.

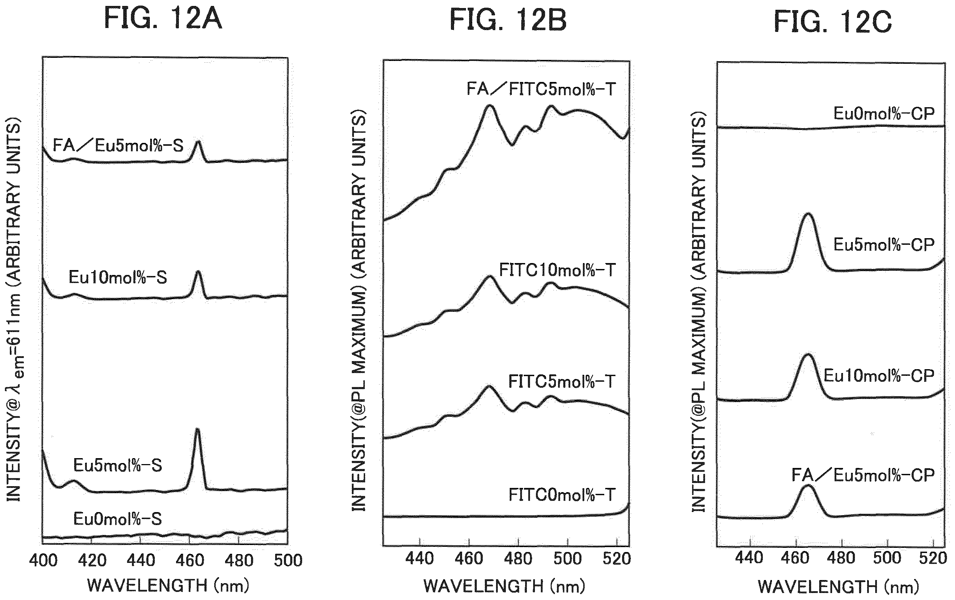

Fig. 12 is a graph showing an excitation spectrum, (a) $Eu^{3+}$-containing silica particles, (b) FITC-containing titania particles, and (c) $Eu^{3+}$-containing calcium phosphate compound particles. In Fig. 12(a) a peak caused by an f-f transition at 465 nm was observed. In Fig. 12(b), at 468, 483, and 493 nm, peaks caused by minus ionized single molecules of the FITC light-emitting substance were observed (the FITC which interacts with the cationic agent ODA (octadecylamine) was introduced individually distributed inside the particles). In Fig. 12(c), a peak caused by an f-f transition at 465 nm was observed.

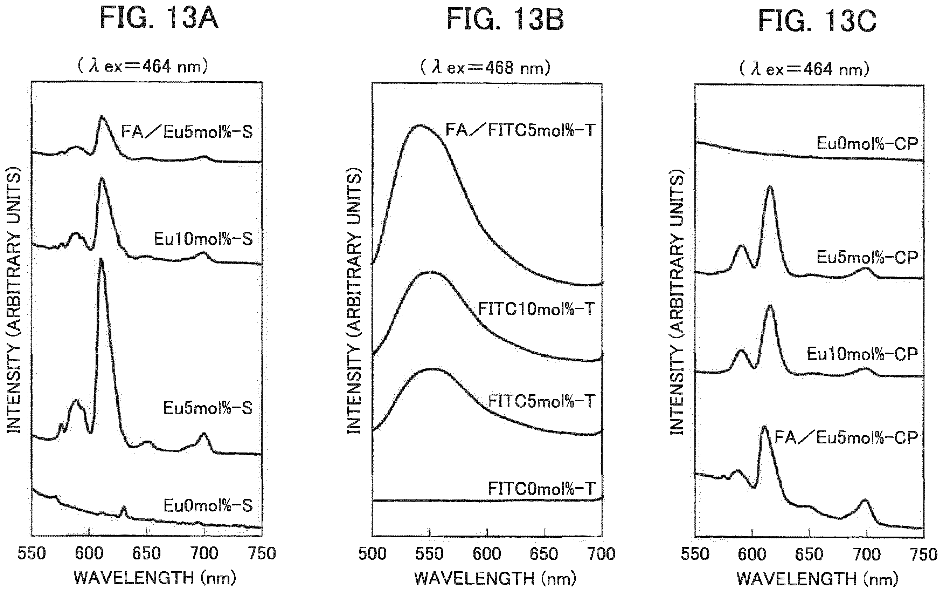

Fig. 13 is a graph showing an emission spectrum, (a) $Eu^{3+}$-containing silica particles, (b) FITC-containing titania particles, and (c) $Eu^{3+}$-containing calcium phosphate compound particles. In Fig. 13(a), peaks caused by a transition from $^5D_0$ to $^7F_0$ of 577 nm, a transition from $^5D_0$ to $^7F_1$ of 585 nm, 590 nm, and 595 nm, a transition from $D_0$ to $^7F_2$ of 611 nm, a transition from $^5D_0$ to $^7F_3$ of 646 nm, and a transition from $^5D_0$ to $^7F_4$ of 577 nm are observed. Ultimately, there was no change in the form and intensity of the light emission spectrum due to the firing or solvent extracting processes (elimination process of the surfactant). From this result, it was thought that the surfactant plays an important role in the approximately uniform dispersion and fixing of the light-emitting substance during the process wherein the particles are nucleated and the crystal growth. In Fig. 13(b), a peak caused by an individually dispersed molecule or a two molecule associated state of the FITC light-emitting substance in the vicinity of 540 nm was observed. Because a peak caused by aggregates was not observed, it was thought to interact with the surfactant and to be present in an approximately uniform distribution. In Fig. 13(c), a fluorescence peak caused by a 4f-4f transition of a light-emitting substance Eu(III) ion; and peaks caused by a transition from $^5D_0$ to $^7F_1$ of 590 nm, a transition from $^5D_0$ to $^7F_2$ of 616 nm, a transition from $^5D_0$ to $^7F_3$ of 652 nm, and a transition from $^5D_0$ to $^7F_4$ of 700 nm were observed. There was no change in light-emission spectral shape or intensity due to the final washing process (surfactant elimination process). From this result, it was thought that the surfactant plays an important role in the approximately uniform dispersion and fixing of the light-emitting substance during the process wherein the particles are nucleated and the crystal growth.

For 5 mol% samples and 10 mol% samples of each light-emitting substance when synthesized and prepared, particles were synthesized using the surfactant, and in the case that the light-emitting substance was approximately uniformly distributed, particles were synthesized by the same experimental method as the present example completely without using surfactant, and the quantum yield was measured in the case that the particles were agglomerated in the light-emitting substance. The quantum yield was determined by a fluorescence spectrum measurement device. Measurements were carried out using an ISF-834 integrating sphere with $\varphi$ 60 mm, and in the measurement of the excitation scattered light, the standard white plate was set and measured in a state with the quartz window plate attached at the reflection position of the integrating sphere. The spectra of the incident light, scattered light, and fluorescence light were measured, their integrated peak intensities were calculated, respectively abbreviated as $I_0$, $I_1$, and $I_2$, and the quantum yield (internal quantum efficiency) $\Phi_{int}$ was calculated by Formula (8). Further, concerning the excitation/fluorescence spectra, the maximum wavelength seen in the excitation/fluorescence spectra of each sample was used.

$$\Phi_{int} = I_2/(I_0 - I_1) \times 100 \qquad \text{Formula (8)}$$

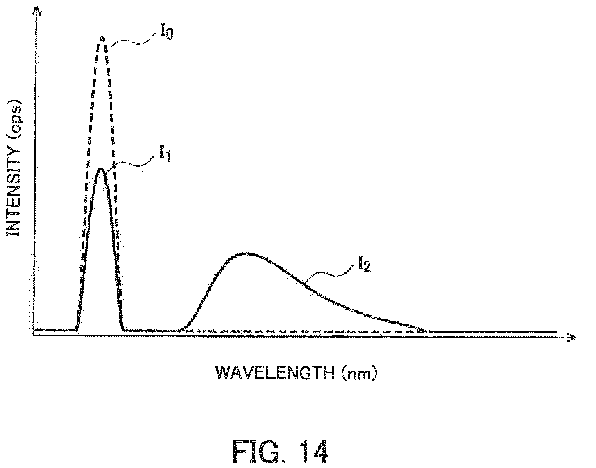

Fig. 14 is a graph showing the intensity spectra of the incident light, scattered light, and fluorescent light. Further, the results of the measured quantum yield as below. The quantum yield in the case of synthesis using the surfactant was higher than the case of not using the surfactant, and it was shown that the surfactant is important in the approximately uniform dispersion of the light-emitting substance (high efficiency light emission).

Eu5mol%-S-synthesis using surfactant: 11.5%
Eu10mol%-S-synthesis using surfactant: 8.3%
Eu5mol%-S-synthesis not using surfactant: 2.5%
Eu10mol%-S-synthesis not using surfactant: 1.3%
FITC5mol%-T-synthesis using surfactant: 19.4%
FITC5mol%-T-synthesis not using surfactant: 13.1%
Eu5mol%-CP-synthesis using surfactant: 7.1%
Eu10mol%-CP-synthesis using surfactant: 4.8%
Eu5mol%-CP-synthesis not using surfactant: 3.6%
Eu10mol%-CP-synthesis not using surfactant: 1.9%

(Viable cell rate of normal cells (fibroblast cells))

Normal cells (NIH3T3 cells) were cultured in a PS flask (dissemination density: 100 x $10^4$ cells/37 $cm^2$) After this, thawing and dissemination were carried out for 7 days, and cells were ablated and separated. The density of the NIH3T3 cells was $(1.97 \pm 0.15) \times 10^5$ cells/mL. Concentration adjustment of the cells was carried out, and 10 vol% FBS (fetal bovine serum) was cultured in DMEM (Dulbecco modification nutrient medium). There were $7.5 \times 10^4$ cells per 1 mL. An amount of 0.9 mL/well was disseminated to a 12 well plate (culture area: 3.8 $cm^2$/well). The disseminated concentration was $1.8 \times 10^4$ cells/$cm^2$. After this, it was cultured (temperature: 37°C, $CO_2$ concentration: 5%, humidity 100%) After 12 hours, FA-Ef:NPS particles were added to 10 vol% DMEM, dispersed, and a concentration of 100 mg/mL was prepared.

Cell proliferation examination was executed by an MTT assay. The MTT assay is a method wherein formazan arising from the reduction of MTT [3-(4,5-dimethylthiazol-2-yl)-2,5-diphenyltetrazolium bromide] by mitochondrial dehydrogenase inside a cell is extracted by an organic solvent, the absorbance at 570 nm is measured, and the viable cell rate is measured. At 24 hours, 48 hours, and 72 hours after dissemination, 100 $\mu$L of MTT reagent (Cat. No. 10009591) was added, and cultured for 3 hours (temperature: 37°C, $CO_2$ concentration: 5%, humidity: 100%). After this, the culture medium was removed, 1 mL of a crystal dissolving solution (Cat. No. 10009593) was added, and stirred (variable mode, 1 min.). The absorbency at 570 nm was measured. The viable cell rate (%) was calculated by the formula below. Viable cell rate (%) = (absorbency of cell which is targeted for an evaluation - absorbency of blank)/(absorbency of cells without added particles - absorbency of blank)

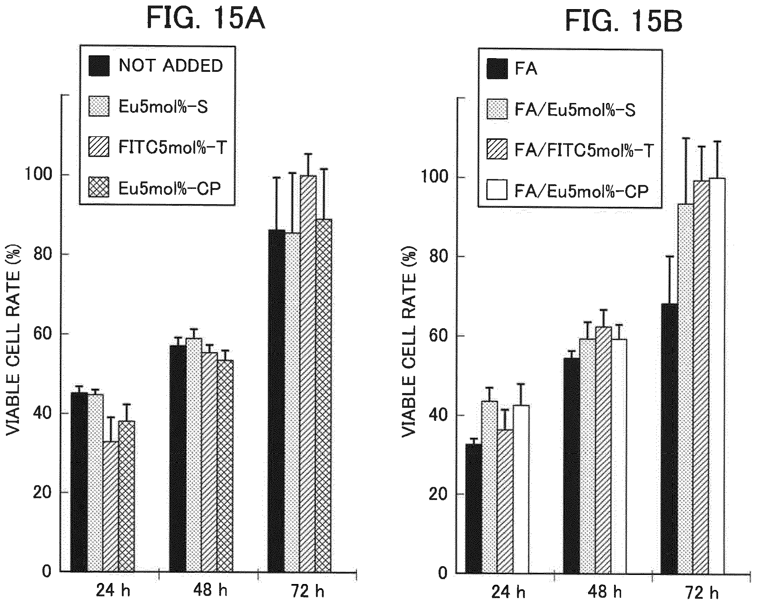

Fig. 15 is a graph showing the results of cytotoxic quantification, (a) light-emitting nanoparticles which are not modified by folic acid bonding to cells, (b) light-emitting nanoparticles which are modified by folic acid bonding to cells. As shown in Fig. 15(a), in all of the folic acid nonmodified (before folic acid modification) particles, the particle additive-free samples, namely, normal cell growth characteristics induced tissue culture polystyrene only same growth characteristics were seen. As shown in Fig. 15(b), in all of the particles after folic acid modification, growth characteristics similar to those of folic acid (FA) only, which does not harm the cell proliferation properties, were seen. As above, the particles of the present embodiment show normal growth characteristics without giving toxicity to the cells.

(Cancer cell imaging and fluorescence intensity measurement)

Hela cancer cells were cultured in a PS flask (dissemination density: $100 \times 10^4$ cells/37 cm$^2$). Thawing and dissemination were carried out for 7 days. The cells were ablated and separated. The Hela concentration was $(0.99 \pm 0.07) \times 10^5$ cells/mL. Concentration adjustment of the cells was carried out, and cultured in DMEM (Dulbecco modification nutrient medium) 10 vol% FBS (fetal bovine serum). There were $7.5 \times 10^4$ cells per 1 mL. An amount of 2.25 mL/PS was disseminated to PS dishes (cultivation area: 9.6 cm$^2$), and the dissemination density was $1.8 \times 10^4$ cells/cm$^2$.

(Microscope observation)

After this, culturing was performed (temperature: 37°C, $CO_2$ concentration: 5%, humidity 100%). After 12 hours, FA-Eu:NPS particles were added to 10 vol% DMEM, dispersed, and the concentration was adjusted to 100 mg/mL.

For living cell imaging, after 3 hours, 24 hours and 48 hours from spraying the particles on the cell surface, the nutrient medium was removed. After this, 1 mL PBS (phosphate buffered saline) was added, and removed (one time). Further, 1 mL of distilled water was added, and removed (one time). Fluorescence intensity measurements were carried out.

Eu$^{3+}$ content: Ex filter: 485 nm $\pm$ 40 nm
Em filter: 590 nm $\pm$ 35 nm
FITC content: Ex filter: 485 nm $\pm$ 40 nm
Em filter: 540 nm $\pm$ 35 nm

Fluorescence microscope observation was carried out only after 24 hours. Further, after cultivation the culture medium was removed and the fluorescence intensity (PL) was measured at specified excitation wavelengths and detection wavelengths, after eliminating the "particles which are not bonded to cells", or "particles which are not taken up into cells" with PBS and distilled water. Therefore, the obtained fluorescence intensities are for light emission caused only by "particles which are bonded to cells", or "particles which are taken up into cells".

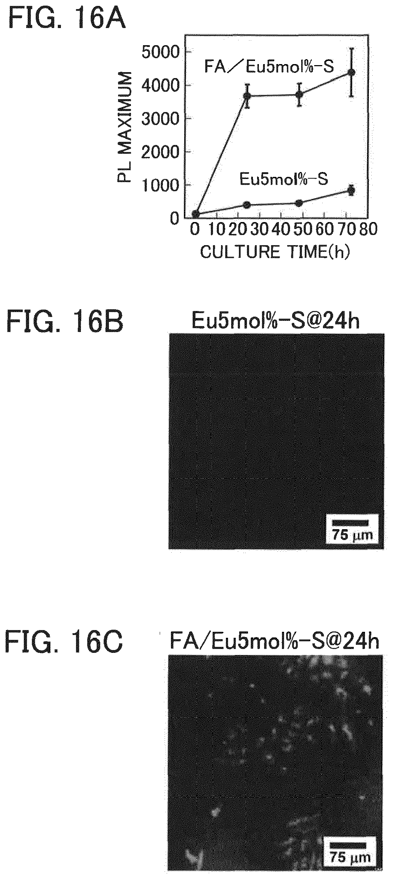

Fig. 16 is, for Eu$^{3+}$-containing silica particles differing by having or not having modification of cell bonding molecules, and (a) is a graph showing the relationship between fluorescent light intensity and culture time, and (b) and (c) are fluorescence imaging images of cells which have taken up the particles. As shown in Fig. 16(a), Eu$^{3+}$-containing silica particles having modification of cell bonding molecules showed a larger increase of fluorescence intensity with respect to culture time than Eu$^{3+}$-containing silica particles not having modification of cell bonding molecules, and after 72 hours showed approximately five times the fluorescence intensity. As shown in Fig. 16(b), in the case of Eu$^{3+}$-containing silica particles not having modification of cell bonding molecules live cell imaging was not possible, but for Eu$^{3+}$-containing silica particles having modification of cell bonding molecules, live cell imaging became possible (Fig. 16(c)). Further, these results show that Eu$^{3+}$-containing silica particles are provided with excellent light-emitting stability and light resistance.

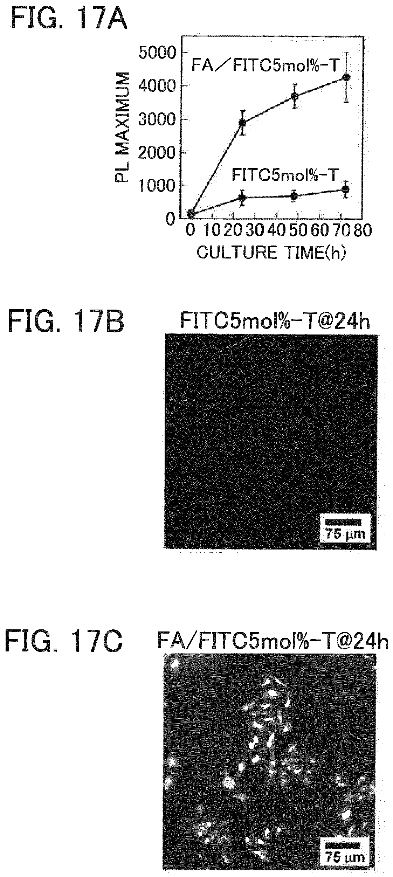

Fig. 17 is, for FITC-containing titania particles differing by having or not having modification of cell bonding molecules, and (a) is a graph showing the relationship between fluorescent light intensity and culture time, and (b) and (c) are fluorescence imaging images of cells which have taken up the particles. As shown in Fig. 17(a), FITC-containing silica particles having modification of cell bonding molecules showed a larger increase of fluorescence intensity with respect to culture time than FITC-containing silica particles not having modification of cell bonding molecules, and after 72 hours showed approximately five times the fluorescence intensity. As shown in Fig. 17(b), in the case of FITC-containing silica particles not having modification of cell bonding molecules live cell imaging was not possible, but for FITC-containing silica particles having modification of cell bonding molecules, live cell imaging became possible (Fig. 17(c)). Further, these results show that FITC-containing silica particles are provided with excellent light-emitting stability and light resistance.

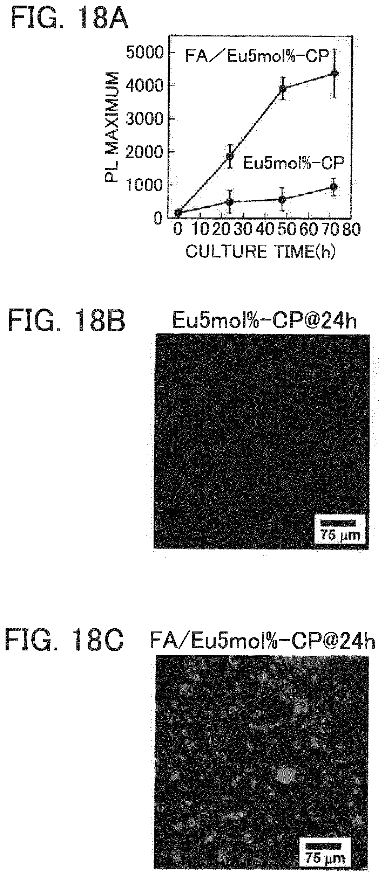

Fig. 18 is, for $Eu^{3+}$-containing calcium phosphate compound particles differing in having or not having modification of cell bonding molecules, and (a) is a graph showing the relationship between fluorescent light intensity and culture time, and (b) and (c) are fluorescence imaging images of cells which have taken up the particles. As shown in Fig. 18(a), $Eu^{3+}$-containing calcium phosphate compound particles having modification of cell bonding molecules showed a larger increase of fluorescence intensity with respect to culture time than $Eu^{3+}$-containing calcium phosphate compound particles not having modification of cell bonding molecules, and after 72 hours showed approximately four times the fluorescence intensity. As shown in Fig. 18(b), in the case of $Eu^{3+}$-containing calcium phosphate compound particles not having modification of cell bonding molecules live cell imaging was not possible, but for $Eu^{3+}$-containing calcium phosphate compound particles having modification of cell bonding molecules, live cell imaging became possible (Fig. 18(c)). Further, these results show that $Eu^{3+}$-containing calcium phosphate compound particles are provided with excellent light-emitting stability and light resistance.

(Measurement Device)

The main measurement device used in the present examples was as follows.

- fluorescence spectrophotometer (manufactured by JASCO Corporation, model name: FP-8500):
  excitation side bandwidth: 10 nm, fluorescence side bandwidth: 10 nm, scanning speed: 200 nm/min, data acquisition interval:
  0.1 nm, response: 1 second, PMT voltage: 350 V. The measurement was carried out for a 20 mg sample via a circular quartz window with a diameter of 16 mm.
- infrared spectrophotometer (manufactured by JASCO Corporation, model name: FT/IR-4100):
  carried out by the KBr method. A powder of the target sample was diluted by a factor of 10 in a KBr powder, and the transmittance (%) was measured. The background was taken as the KBr powder, the integration number was set to 100, and the resolution was set to 2.0 $cm^{-1}$.
- scanning electron microscope (manufactured by Hitachi High Technologies K.K., model name: SU8000) [used with titania particle type (T)]:
  Observed with conditions of FE voltage 5 kV, current 10 $\mu$A. An ethanol suspension of nanoparticles adjusted to 0.01 wt% was dripped onto a silicon base plate, dried and observed.
- transmission electron microscope (manufactured by Hitachi High Technologies K.K. model name: HT 7700) [used for silica (S) particle system and calcium phosphate compound particle system (CP)]:
  An ethanol suspension of nanoparticles adjusted to 0.01 wt% was dripped onto a copper grid (made by Okenshoji Co., Ltd., product name: carbon/formvar film) coated with carbon. The dripped grid was dried under a nitrogen atmosphere for 24 hours in a desiccator, and observed with an acceleration voltage of 120 kV.
- fluorescence microscope (made by Olympus, device name: CKX41):
  exposure time was set to 100 msec, sensitivity was set to ISO 400. Further, a light source made by Olympus, device name: U-RFLT50, was used. The sample was irradiated via a die lock mirror with the specified wavelength bandwidth (patent explanatory material PDF file page 21), and the emitted light was detected via the die lock mirror and the absorption filter.
- powder X-ray diffraction (made by Rigaku Corporation, device name: Smart Lab):
  with measurement conditions of, X-ray source: CuK $\alpha$ line source ($\lambda$: 1.5418 Å), output: 40 kV/30 mA, scan speed: 5.0°/min, sampling width: 0.01°, measurement mode: continuous. The diffraction line position, diffraction angle, and half value width were obtained by software (made by Rigaku Corporation, software name: PDXL).
- flourescence X-ray analysis (made by Rigaku Corporation, device name: ZSX Primus II):
  A pellet with a diameter of 10 mm of the sample powder was produced using an oil pressure hand press. The measurement was analyzed using the software belonging to the device (made by Rigaku Corporation, software name: EZ scan program).

EXPLANATION OF REFERENCE NUMERALS

1... light-emitting nanoparticle, 2...matrix material, 3... light-emitting substance, 4...OH group or amino group, 5...peptide bond, 6... cell bonding molecule, 7... cell bonding molecule-modified light-emitting nanoparticle , 10...cancer cell, 11...receptor.

**Claims**

1. A light-emitting nanoparticle comprising a matrix material, and a light-emitting substance included in the matrix material, wherein the matrix material comprises at least one cationic element selected from the group consisting of Ti, Si, Ca, Al and Zr and surfactant molecule, and at least one anionic element selected from the group consisting of O and P, preferably comprising at least one selected from the group consisting of $TiO_2$, $Ca_{10}(PO_4)_6(OH)_2$, $Al_2O_3$, and $ZrO_2$.

2. A light-emitting nanoparticle according to claim 1,
   wherein the matrix material comprises at least one cationic element selected from the group consisting of Ti, Ca, Al and Zr, preferably comprising at least one selected from the group consisting of $TiO_2$, $Ca_{10}(PO_4)_6(OH)_2$, $Al_2O_3$, and $ZrO_2$.

3. A light-emitting nanoparticle according to claim 1 or 2,
   wherein a concentration of the light-emitting substance in the matrix material is a concentration whereby an average distance between the light-emitting substance is 1.2 nm or more and 10 nm or less.

4. A light-emitting nanoparticle according to claim 3, wherein the average distance between light-emitting substance is calculated according to the following formula:

$$\underline{D = \{V_2 \bullet 3/(4\pi)\}^{1/3}}$$

wherein $V_2$ is an occupied volume of 1 molecule of light-emitting substance ($nm^3$/1 molecule of light-emitting substance) and is calculated according to the following formula:

$$V2 = V1/X2$$

wherein $V_1$ is an average one particle volume ($nm^3$/1 particle) and is calculated according to the following formula:

$$V1 = (4\pi/3) \bullet (R/2)^3$$

wherein R is an average particle diameter (nm), and $X_2$ is a light-emitting substance number contained in the light-emitting nanoparticle (molecule number/1 particle), and when B is taken as an inorganic molecule number per light-emitting substance number (molecule number/substance number), is calculated according to the following formula:

$$\underline{X_2 = X_1 \bullet B}$$

wherein $X_1$ is an inorganic molecule number contained in the light-emitting nanoparticle (molecule number/1 particle) and B is the inorganic molecule number per light-emitting substance number (molecule number/substance number).

5. A light-emitting nanoparticle according to any one of claims 1 to 3, comprising as the light-emitting substance, at least one of a rare-earth ion, and an organic light-emitting dye at 1 mol% to 5 mol% with respect to the cationic element.

6. A light-emitting nanoparticle according to any one of claims 1 to 5, comprising a fluorescein-based dye molecule as an organic light-emitting dye.

7. A light-emitting nanoparticle according to any one of claims 1 to 6, wherein the rare earth ion comprises trivalent Eu.

8. A light-emitting nanoparticle according to any one of claims 1 to 7, wherein a contained concentration of the rare earth ion is 1 mmol% to 10 mol% with respect to the cationic element.

9. A light-emitting nanoparticle according to any one of claims 1 to 8, wherein an average particle diameter of the light-emitting nanoparticles is 10 nm to 500 nm.

10. A light-emitting nanoparticle according to any one of claims 1 to 9, wherein a surface is provided with a micropore with a pore diameter of 0.1 to 10 nm.

11. A light-emitting nanoparticle according to any one of claims 1 to 10, wherein a hydroxyl group and/or amino group bonded to the cationic element is formed at a surface.

12. A light-emitting nanoparticle according to any one of claims 1 to 11, wherein a surface is modified by a cell bonding molecule consisting of one or more selected from the group consisting of HER2 antibody, antibodies selectively binding to human epidermal growth factor receptor, cancer-specific antibodies, phosphorylation protein antibodies, folic acid, antibodies selectively binding to folic acid receptor β, vascular endothelial cell-specific antibodies, tissue-specific antibodies, transferrin, transferring-bonding peptide, and proteins having affinity to sugar chains.

13. A light-emitting nanoparticle according to any one of claims 1 to 12, wherein an excitation wavelength and a light emission wavelength are in the visible light region.

14. A light-emitting nanoparticle according to any one of claims 1 to 13, used for bioimaging and/or as a therapeutic agent and which supports a drug at a pore of a surface.

**Patentansprüche**

1. Lichtemittierender Nanopartikel, der ein Matrixmaterial und eine in dem Matrixmaterial enthaltene lichtemittierende Substanz umfasst, wobei das Matrixmaterial mindestens ein kationisches Element, ausgewählt aus der Gruppe bestehend aus Ti, Si, Ca, Al und Zr, und ein oberflächenaktives Molekül, und mindestens ein anionisches Element, ausgewählt aus der Gruppe, bestehend aus O und P, umfasst, vorzugsweise mindestens eines, ausgewählt aus der Gruppe, bestehend aus $TiO_2$, $Ca_{10}(PO_4)_6 (OH)_2$, $Al_2O_3$, und $ZrO_2$.

2. Lichtemittierender Nanopartikel nach Anspruch 1, wobei das Matrixmaterial mindestens ein kationisches Element umfasst, das aus der Gruppe ausgewählt ist, die aus Ti, Ca, Al und Zr besteht, und vorzugsweise mindestens eines umfasst, das aus der Gruppe ausgewählt ist, die aus $TiO_2$, $Ca_{10}(PO_4)_6(OH)_2$, $Al_2O_3$ und $ZrO_2$ besteht.

3. Lichtemittierender Nanopartikel nach Anspruch 1 oder 2, wobei die Konzentration der lichtemittierenden Substanz in dem Matrixmaterial eine Konzentration ist, bei der der durchschnittliche Abstand zwischen der lichtemittierenden Substanz 1,2 nm oder mehr und 10 nm oder weniger beträgt.

4. Lichtemittierender Nanopartikel nach Anspruch 3, wobei der durchschnittliche Abstand zwischen der lichtemittierenden Substanz nach der folgenden Formel berechnet wird:

$$\underline{D = \{V_2 \cdot 3/(4\pi)\}^{1/3}}$$

wobei $V_2$ das belegte Volumen von 1 Molekül der lichtemittierenden Substanz ist ($nm^3$/1 Molekül der lichtemittierenden Substanz) und nach der folgenden Formel berechnet wird:

$$V2 = V1/X2$$

wobei $V_1$ ein durchschnittliches Partikelvolumen ($nm^3$/1 Partikel) ist und nach der folgenden Formel berechnet wird:

$$V1 = (4\pi/3) \cdot (R/2)^3$$

wobei R ein durchschnittlicher Teilchendurchmesser (nm) ist,
und $X_2$ eine in dem lichtemittierenden Nanopartikel enthaltene lichtemittierende Substanzzahl (Molekülzahl/1 Partikel) ist, und wenn B als anorganische Molekülzahl pro lichtemittierende Substanzzahl (Molekülzahl/Substanzzahl) genommen wird, nach der folgenden Formel berechnet wird:

$$\underline{X_2 = X_1 \cdot B}$$

wobei $X_1$ eine anorganische Molekülzahl ist, die in dem lichtemittierenden Nanopartikel enthalten ist (Molekülzahl/1 Partikel), und B die anorganische Molekülzahl pro lichtemittierender Substanzzahl ist (Molekülzahl/Substanzzahl).

5. Lichtemittierender Nanopartikel nach einem der Ansprüche 1 bis 3, der als lichtemittierende Substanz mindestens eines von einem Seltenerd-Ion und einem organischen lichtemittierenden Farbstoff in einer Menge von 1 Mol-% bis 5 Mol-%, bezogen auf das kationische Element, enthält.

6. Lichtemittierender Nanopartikel nach einem der Ansprüche 1 bis 5, umfassend ein Farbstoffmolekül auf Fluoresceinbasis als organischen lichtemittierenden Farbstoff.

7. Lichtemittierender Nanopartikel nach einem der Ansprüche 1 bis 6, wobei das Seltenerdion dreiwertiges Eu umfasst.

8. Lichtemittierender Nanopartikel nach einem der Ansprüche 1 bis 7, wobei die enthaltene Konzentration des Seltenerdions 1 mmol% bis 10 mol%, bezogen auf das kationische Element, beträgt.

9. Lichtemittierender Nanopartikel nach einem der Ansprüche 1 bis 8, wobei der durchschnittliche Teilchendurchmesser der lichtemittierenden Nanopartikel 10 nm bis 500 nm beträgt.

10. Lichtemittierender Nanopartikel nach einem der Ansprüche 1 bis 9, wobei eine Oberfläche mit einer Mikropore mit einem Porendurchmesser von 0,1 bis 10 nm versehen ist.

11. Lichtemittierender Nanopartikel nach einem der Ansprüche 1 bis 10, wobei eine an das kationische Element gebundene Hydroxylgruppe und/oder Aminogruppe an einer Oberfläche gebildet ist.

12. Lichtemittierender Nanopartikel nach einem der Ansprüche 1 bis 11, wobei eine Oberfläche durch ein zellbindendes Molekül modifiziert ist, das aus einem oder mehreren aus der Gruppe bestehend aus HER2-Antikörper, Antikörper, die selektiv an den humanen epidermalen Wachstumsfaktorrezeptor binden, krebsspezifische Antikörper, Phosphorylierungsprotein-Antikörper, Folsäure, Antikörper, die selektiv an den Folsäurerezeptor $\beta$ binden, vaskuläre endotheliale Zell-spezifische Antikörper, gewebespezifische Antikörper, Transferrin, übertragendes Bindungspeptid und Proteine mit Affinität zu Zuckerketten besteht.

13. Lichtemittierender Nanopartikel nach einem der Ansprüche 1 bis 12, wobei eine Anregungswellenlänge und eine Lichtemissionswellenlänge im Bereich des sichtbaren Lichts liegen.

14. Lichtemittierender Nanopartikel nach einem der Ansprüche 1 bis 13, der für die Biobildgebung und/oder als therapeutisches Mittel verwendet wird und das ein Arzneimittel an einer Pore einer Oberfläche trägt.

**Revendications**

1. Nanoparticule luminescente comprenant un matériau de matrice et une substance luminescente incluse dans le matériau de matrice, dans laquelle le matériau de matrice comprend au moins un élément cationique sélectionné dans le groupe consistant en Ti, Si, Ca, Al et Zr et une molécule tensioactive, et au moins un élément anionique sélectionné dans le groupe consistant en O et P, comprenant de préférence au moins un élément sélectionné dans le groupe consistant en $TiO_2$, $Ca_{10}(PO_4)_6(OH)_2$, $Al_2O_3$, et $ZrO_2$.

2. Nanoparticule luminescente selon la revendication 1, dans laquelle le matériau de matrice comprend au moins un élément cationique sélectionné dans le groupe consistant en Ti, Ca, Al et Zr, comprenant de préférence au moins un élément sélectionné parmi le groupe consistant en $TiO_2$, $Ca_{10}(PO_4)_6 (OH)_2$, $Al_2O_3$, et $ZrO_2$.

**3.** Nanoparticule luminescente selon la revendication 1 ou 2, dans laquelle une concentration de la substance luminescente dans le matériau de matrice est une concentration telle qu'une distance moyenne entre les substances luminescentes est de 1,2 nm ou plus et de 10 nm ou moins.

**4.** Nanoparticule luminescente selon la revendication 3, dans laquelle la distance moyenne entre les substances luminescentes est calculée selon la formule suivante :

$$\underline{D \;=\; \{V_2 \bullet 3/(4\,\pi)\}^{1/3}}$$

dans laquelle $V_2$ est un volume occupé par une molécule de substance luminescente ($nm^3$/1 molécule de substance luminescente) et est calculé selon la formule suivante :

$$V2 \;=\; V1/X2$$

dans laquelle V1 est un volume moyen d'une particule ($nm^3$/1 particule) et est calculé selon la formule suivante :

$$V1 \;=\; (4\pi/3)\bullet(R/2)^3$$

dans laquelle R est un diamètre moyen de particule (nm),
et $X_2$ est un nombre de substances luminescentes contenues dans la nanoparticule luminescente (nombre de molécules/1 particule), et lorsque B est pris en tant que nombre de molécules inorganiques par nombre de substances luminescentes (nombre de molécules/nombre de substances), est calculé selon la formule suivante :

$$\underline{X_2 \;=\; X_1 \bullet B}$$

dans laquelle $X_1$ est un nombre de molécules inorganiques contenues dans la nanoparticule luminescente (nombre de molécules/1 particule) et B est le nombre de molécules inorganiques par nombre de substances luminescentes (nombre de molécules/nombre de substances).

**5.** Nanoparticule luminescente selon l'une quelconque des revendications 1 à 3, comprenant en tant que substance luminescente, au moins un parmi un ion de terre rare et un colorant organique luminescent à 1 % en mole à 5 % en mole par rapport à l'élément cationique.

**6.** Nanoparticule luminescente selon l'une quelconque des revendications 1 à 5, comprenant une molécule de colorant à base de fluorescéine en tant que colorant organique luminescent.

**7.** Nanoparticule luminescente selon l'une quelconque des revendications 1 à 6, dans laquelle l'ion de terre rare comprend de l'Eu trivalent.

**8.** Nanoparticule luminescente selon l'une quelconque des revendications 1 à 7, dans laquelle une concentration contenue de l'ion de terre rare est de 1 % en mmole à 10 % en mole par rapport à l'élément cationique.

**9.** Nanoparticule luminescente selon l'une quelconque des revendications 1 à 8, dans laquelle un diamètre moyen de particule des nanoparticules luminescentes est de 10 nm à 500 nm.

**10.** Nanoparticule luminescente selon l'une quelconque des revendications 1 à 9, dans laquelle une surface est pourvue d'un micropore ayant un diamètre de pore de 0,1 à 10 nm.

**11.** Nanoparticule luminescente selon l'une quelconque des revendications 1 à 10, dans laquelle un groupe hydroxyle et/ou un groupe amino liés à l'élément cationique est formé sur une surface.

**12.** Nanoparticule luminescente selon l'une quelconque des revendications 1 à 11, dans laquelle une surface est modifiée par une molécule de liaison aux cellules constituée d'un ou de plusieurs éléments sélectionnés dans le groupe consistant en un anticorps HER2, les anticorps se liant sélectivement au récepteur de facteur de croissance épidermique humain, les anticorps spécifiques d'un cancer, les anticorps de protéine de phosphorylation, l'acide folique, les anticorps se liant sélectivement au récepteur β de l'acide folique, les anticorps spécifiques des cellules endothéliales vasculaires, les anticorps spécifiques de tissus, la transferrine, un peptide se liant à la transferrine, et les protéines ayant une affinité pour les chaînes de sucre.

13. Nanoparticule luminescente selon l'une quelconque des revendications 1 à 12, dans laquelle une longueur d'onde d'excitation et une longueur d'onde d'émission de lumière se situent dans la région de la lumière visible.

14. Nanoparticule luminescente selon l'une quelconque des revendications 1 à 13, utilisée pour la bioimagerie et/ou comme agent thérapeutique et qui supporte un médicament au niveau d'un pore d'une surface.

FIG. 1

FIG. 2

EP 3 438 227 B1

FIG. 3A

Eu10mol%-S

FIG. 3B

FITC10mol%-T

FIG. 3C

Eu10mol%-CP

2 nm

2 nm

2 nm

## FIG. 4A

## FIG. 4B

## FIG. 4C

## FIG. 5A

Eu0mol%–S

## FIG. 5B

Eu5mol%–S

## FIG. 5C

Eu10mol%–S

FIG. 6A  Eu0mol%-S

FIG. 6B  Eu5mol%-S

FIG. 6C  Eu10mol%-S

FIG. 6D

FIG. 6E

FIG. 6F

# EP 3 438 227 B1

**FIG. 7A**

FITC0mol%-T

$D$ = 417 nm
$CV$ = 10.4 %

**FIG. 7B**

FITC5mol%-T

$D$ = 418 nm
$CV$ = 8.1 %

**FIG. 7C**

FITC10mol%-T

$D$ = 423 nm
$CV$ = 8.4 %

32

## Eu0mol%–CP  FIG. 8A

## Eu5mol%–CP  FIG. 8B

## Eu10mol%–CP  FIG. 8C

FIG. 9A

FIG. 9B

FIG. 9C

34

# FIG. 10

EP 3 438 227 B1

FIG. 11A

FIG. 11B

FIG. 11C

# FIG. 12A

INTENSITY@$\lambda_{em}$=611nm (ARBITRARY UNITS)

FA／Eu5mol%-S

Eu10mol%-S

Eu5mol%-S

Eu0mol%-S

WAVELENGTH (nm)

400  420  440  460  480  500

# FIG. 12B

INTENSITY(@PL MAXIMUM) (ARBITRARY UNITS)

FA／FITC5mol%-T

FITC10mol%-T

FITC5mol%-T

FITC0mol%-T

WAVELENGTH (nm)

440  460  480  500  520

# FIG. 12C

INTENSITY(@PL MAXIMUM) (ARBITRARY UNITS)

Eu0mol%-CP

Eu5mol%-CP

Eu10mol%-CP

FA／Eu5mol%-CP

WAVELENGTH (nm)

440  460  480  500  520

FIG. 13A  FIG. 13B  FIG. 13C

FIG. 14

# FIG. 15A

# FIG. 15B

EP 3 438 227 B1

FIG. 16A

FIG. 16B

Eu5mol%-S@24h

FIG. 16C

FA/Eu5mol%-S@24h

## FIG. 17A

## FIG. 17B

FITC5mol%-T@24h

## FIG. 17C

FA/FITC5mol%-T@24h

FIG. 18A

FIG. 18B    Eu5mol%-CP@24h

FIG. 18C    FA/Eu5mol%-CP@24h

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010133887 A
- JP 2009190976 A
- JP 2009107106 A
- JP 2013014651 A
- WO 2009066548 A
- JP 2013057037 A
- WO 2005023961 A

### Non-patent literature cited in the description

- Selective molecular imaging of viable cancer cells with pH-activatable fluorescence probes. *Nature Medicine,* 2009, vol. 15, 104
- Quantum Dot Bioconjugates for Ultrasensitive Nonisotopic Detection. *Science,* 1998, vol. 281, 2016
- Nucleic Acid-Passivated Semiconductor Nanocrystals: Biomolecular Templating of Form and Function. *Accounts of Chemical Research,* 2010, vol. 43, 173
- Multimodal-Luminescence Core-Shell Nanocomposites for Targeted Imaging of Tumor Cells. *Chem. Eur. J.,* 2009, vol. 15, 3577
- Synthesis of Luminescent Nanoporous Silica Spheres Functionalized with Folic Acid for Targeting to Cancer Cells. *Inorganic Chemistry,* 12 June 2014, vol. 53 (13), 6817-6827
- Large-Scale Synthesis of Water-Soluble Luminescent Hydroxyapatite Nanorods for Security Printing. *Journal of Colloid and Interface Science,* 01 February 2016, vol. 468, 300-306
- Synthesis of Cytocompatible Luminescent Titania/Fluorescin Hybrid Nanoparticles. *ACS Applied materials & Interfaces,* 25 April 2014, vol. 6 (9), 6825-6834
- Synthesis and Luminescence Properties of Eu(III)-doped Nanoporous Silica Spheres. Journal of Colloid and Interface Science. Academic Press, Inc, 17 July 2011, vol. 363, 456-464
- Efficient Synthesis of Eu(III)-Containing Nanoporous Silicas. Materials Letters. Elsevier, 03 April 2011, vol. 65, 2287-2290
- In Vitro Targeting of Cancer Cells with Luminescent Nanoporous Silica Spheres. Transactions on Gigaku. Nagaoka University of technology, 30 June 2012, vol. 1, 1-8
- Persistent Luminescence of Eu, Mn, Dy-doped Calcium Phosphates for in-Vivo Optical Imaging. Journal of Luminescence. Elsevier BV North-Holland, 29 July 2015, vol. 170, 460-466
- Self-Activated Luminescent and Mesoporous Strontium Hydroxyapatite Nanorods for Drug Delivery. Biomaterials. Elsevier Science Publishers BV, 01 February 2010, vol. 31, 3374-3383
- **M. LAUBE ; F. RAUCH ; C. OTTERMANN ; O. ANDERSON ; K. BANGE.** *Nucl. Instrum. Methods Phys. Res., Sect. B,* 1996, vol. 113, 288-292
- **C. R. OTTERMANNN ; K. BANGE.** *Thin Solid Films,* 1996, vol. 286, 32-34
- **D. MERGEL ; D. BUSCHENDORF ; S. EGGERT ; R. GRAMMES ; B. SAMSET.** *Thin Solid Films,* 2000, vol. 371, 218-224
- **D MERGEL.** *Thin Solid Films,* 2001, vol. 397, 216-222
- **V. V. HOANG ; H. ZUNG ; N. H. B. TRONG.** *Eur. Phys. J. D,* 2007, vol. 44, 515-524